# EUROPÄISCHE PATENTSCHRIFT

(11) **EP 1 204 430 B1**
(45) Veröffentlichungstag und Bekanntmachung des Hinweises auf die Patenterteilung: **04.11.2009**
(21) Anmeldenummer: 00956220.8
(22) Anmeldetag: 20.07.2000
(51) Int. Cl.: A61K 47/48, A61K 49/00, A61P 43/00

(54) **KONJUGATE UND VERFAHREN ZU DEREN HERSTELLUNG SOWIE DEREN VERWENDUNG ZUM TRANSPORT VON MOLEKÜLEN ÜBER BIOLOGISCHE MEMBRANEN**
CONJUGATES AND METHODS FOR THE PRODUCTION THEREOF, AND THEIR USE FOR TRANSPORTING MOLECULES VIA BIOLOGICAL MEMBRANES
CONJUGATS ET PROCEDES PERMETTANT DE LES PREPARER, ET LEUR UTILISATION POUR TRANSPORTER DES MOLECULES PAR L'INTERMEDIAIRE DE MEMBRANES BIOLOGIQUES

(30) Priorität: 28.07.1999 DE 19935302
(43) Veröffentlichungstag der Anmeldung: 15.05.2002
(73) Patentinhaber: Sanofi-Aventis Deutschland GmbH, 65929 Frankfurt am Main (DE)
(72) Erfinder: UHLMANN, Eugen, D-61479 Glashütten (DE); GREINER, Beate, D-65812 Bad Soden (DE); UNGER, Eberhard, D-07751 Jena-Cospeda (DE); GOTHE, Gislinde, D-07751 Jena-Cospeda (DE); SCHWERDEL, Marc, D-07745 Jena (DE)
(86) Internationale Anmeldenummer: PCT/EP2000/006936
(87) Internationale Veröffentlichungsnummer: WO 2001/008707

(56) Entgegenhaltungen:
- EP-A- 0 962 497
- WO-A-95/06659
- LUEHRSEN K R ET AL: "High-density hapten labeling and HRP conjugation of oligonucleotides for use as in situ hybridization probes to detect mRNA targets in cells and tissues." JOURNAL OF HISTOCHEMISTRY AND CYTOCHEMISTRY, (2000 JAN) 48 (1) 133-45. , XP001005917
- PRAKASH T P ET AL: "2'-O- 2-N,N-(DIALKYL)AMINOOXYETHYL - MODIFIED ANTISENSE OLIGONUCLEOTIDES" ORGANIC LETTERS,ACS, WASHINGTON, DC,US, Bd. 2, Nr. 25, 14. Dezember 2000 (2000-12-14), Seiten 3995-3998, XP001002277 ISSN: 1523-7060
- MANOHARAN M: "2'-CARBOHYDRATE MODIFICATIONS IN ANTISENSE OLIGONUCLEOTIDE THERAPY:IMPORTANCE OF CONFORMATION, CONFIGURATION AND CONJUGATION" BIOCHIMICA ET BIOPHYSICA ACTA,AMSTERDAM,NL, Bd. 1489, 1999, Seiten 117-130, XP000979474 ISSN: 0006-3002
- WHITTEMORE N. A. ET AL: "Synthesis and Electrochemistry of anthraquinone-oligodeoxynucleotide conjugates" BIOCONJUGATE CHEM, Bd. 10, 2. Mai 1999 (1999-05-02), Seiten 261-270, XP001002553
- RAIT A. ET AL: "3' end conjugates of minimally phosphorothioate-protected oligonucleotides with 1-O-hexadecylglycerol: synthesis and anti-ras activity in radiation resitant cells" BIOCONJUGATE CHEM, Bd. 11, 1. August 2000 (2000-08-01), Seiten 153-160, XP001002511
- STARKE D ET AL: "Bile acid-oligodeoxynucleotide conjugates: synthesis and liver excretion in rats" BIOORGANIC & MEDICINAL CHEMISTRY LETTERS,OXFORD,GB, Bd. 11, Nr. 7, 9. April 2001 (2001-04-09), Seiten 945-949, XP004232530 ISSN: 0960-894X

### SEQUENZPROTOKOLL

<110> Aventis Pharma Deutschland GmbH
<120> Konjugate und Verfahren zu deren Herstellung sowie deren Verwendung zum Transport von Molekülen über biologische Membranen
<130> 1999/L045
<140>
   <141>
<150> 19935302.6
   <151> 1999-07-28
<160> 63
<170> PatentIn Ver. 2.1
<210> 1
   <211> 16
   <212> DNA
   <213> Künstliche Sequenz
<220>
   <223> Beschreibung der künstlichen Sequenz: modified Oligonucleotide
<220>
   <221> misc_feature
   <222> (1)..(16)
<400> 1
   dgcgacgcca tgacgg 16
<210> 2
   <211> 13
   <212> DNA
   <213> Künstliche Sequenz
<220>
   <223> Beschreibung der künstlichen Sequenz: modified Oligonucleotide
<220>
   <221> misc_feature
   <222> (1)..(13)
<400> 2
   dcgacgccat gac 13
<210> 3
   <211> 13
   <212> DNA
   <213> Künstliche Sequenz
<220>
   <223> Beschreibung der künstlichen Sequenz: modified Oligonucleotide
<220>
   <221> misc_feature
   <222> (1)..(13)
<400> 3
   datgacggaa ttc 13
<210> 4
   <211> 12
   <212> DNA
   <213> Künstliche Sequenz
<220>
   <223> Beschreibung der künstlichen Sequenz: Oligonucleotide
<220>
   <221> misc_feature
   <222> (1)..(12)
<400> 4
   dtattccgtc at 12
<210> 5
   <211> 2
   <212> DNA
   <213> Künstliche Sequenz
<220>
   <223> Beschreibung der künstlichen Sequenz: Oligonucleotide
<220>
   <221> misc_feature
   <222> (1)..(2)
<400> 5
   da 2
<210> 6
   <211> 2
   <212> DNA
   <213> Künstliche Sequenz
<220>
   <223> Beschreibung der künstlichen Sequenz: Oligonucleotide
<220>
   <221> misc_feature
   <222> (1)..(2)
<400> 6
   da 2
<210> 7
   <211> 2
   <212> DNA
   <213> Künstliche Sequenz
<220>
   <223> Beschreibung der künstlichen Sequenz: Oligonucleotide
<220>
   <221> misc_feature
   <222> (1)..(2)
<400> 7
   da 2
<210> 8
   <211> 12
   <212> DNA
   <213> Künstliche Sequenz
<220>
   <223> Beschreibung der künstlichen Sequenz: modified Oligonucleotide
<220>
   <221> misc_feature
   <222> (1)..(12)
<400> 8
   ttccatggtg gc 12
<210> 9
   <211> 12
   <212> DNA
   <213> Künstliche Sequenz
<220>
   <223> Beschreibung der künstlichen Sequenz: modified Oligonucleotide
<220>
   <221> misc_feature
   <222> (1)..(12)
<400> 9
   ttcactgtgg gc 12
<210> 10
   <211> 12
   <212> DNA
   <213> Künstliche Sequenz
<220>
   <223> Beschreibung der künstlichen Sequenz: modified Oligonucleotide
<220>
   <221> misc_feature
   <222> (1)..(12)
<400> 10
   tggcgccggg cc 12
<210> 11
   <211> 12
   <212> DNA
   <213> Künstliche Sequenz
<220>
   <223> Beschreibung der künstlichen Sequenz: modified Oligonucleotide
<220>
   <221> misc_feature
   <222> (1)..(12)
<400> 11
   tgccggccgg gc 12
<210> 12
   <211> 21
   <212> DNA
   <213> Künstliche Sequenz
<220>
   <223> Beschreibung der künstlichen Sequenz: Oligonucleotide
<220>
   <221> misc_feature
   <222> (1)..(21)
<400> 12
   gcgtttgctc ttcttcttgc g 21
<210> 13
   <211> 20
   <212> DNA
   <213> Künstliche Sequenz
<220>
   <223> Beschreibung der künstlichen Sequenz: oligonucleotide
<220>
   <221> misc_feature
   <222> (1)..(20)
<400> 13
   acacccaatt ctgaaaatgg 20
<210> 14
   <211> 20
   <212> DNA
   <213> Künstliche Sequenz
<220>
   <223> Beschreibung der künstlichen Sequenz: Oligonucleotide
<220>
   <221> misc_feature
   <222> (1)..(20)
<400> 14
   aggtccctgt tcgggcgcca 20
<210> 15
   <211> 20
   <212> DNA
   <213> Künstliche Sequenz
<220>
   <223> Beschreibung der künstlichen Sequenz: oligonucleotide
<220>
   <221> misc_feature
   <222> (1)..(20)
<400> 15
   gcggggctcc atgggggtcg 20
<210> 16
   <211> 15
   <212> DNA
   <213> Künstliche Sequenz
<220>
   <223> Beschreibung der künstlichen Sequenz: Oligonucleotide
<220>
   <221> misc_feature
   <222> (1)..(15)
<400> 16
   cagctgcaac ccagc 15
<210> 17
   <211> 11
   <212> DNA
   <213> Künstliche Sequenz
<220>
   <223> Beschreibung der künstlichen Sequenz: Oligonucleotide
<220>
   <221> misc_feature
   <222> (1)..(11)
<400> 17
   tattccgtca t 11
<210> 18
   <211> 22
   <212> DNA
   <213> Künstliche Sequenz
<220>
   <223> Beschreibung der künstlichen Sequenz: Oligonucleotide
<220>
   <221> misc_feature
   <222> (1)..(22)
<400> 18
   ttccgtcatc gctcctcagg gg 22
<210> 19
   <211> 15
   <212> DNA
   <213> Künstliche Sequenz
<220>
   <223> Beschreibung der künstlichen Sequenz: Oligonucleotide
<220>
   <221> misc_feature
   <222> (1)..(15)
<400> 19
   ggctgccatg gtccc 15
<210> 20
   <211> 21
   <212> DNA
   <213> Künstliche Sequenz
<220>
   <223> Beschreibung der künstlichen Sequenz: Oligonucleotide
<220>
   <221> misc_feature
   <222> (1)..(21)
<400> 20
   ggctgctgga gcggggcaca c 21
<210> 21
   <211> 15
   <212> DNA
   <213> Künstliche Sequenz
<220>
   <223> Beschreibung der künstlichen Sequenz: Oligonucleotide
<220>
   <221> misc_feature
   <222> (1)..(15)
<400> 21
   aacgttgagg ggcat 15
<210> 22
   <211> 18
   <212> DNA
   <213> Künstliche Sequenz
<220>
   <223> Beschreibung der künstlichen Sequenz: Oligonucleotide
<220>
   <221> misc_feature
   <222> (1)..(18)
<400> 22
   gtgccggggt cttcgggc 18
<210> 23
   <211> 17
   <212> DNA
   <213> Künstliche Sequenz
<220>
   <223> Beschreibung der künstlichen Sequenz: Oligonucleotide
<220>
   <221> misc_feature
   <222> (1)..(17)
<400> 23
   cgagaacatc atcgtgg 17
<210> 24
   <211> 21
   <212> DNA
   <213> Künstliche Sequenz
<220>
   <223> Beschreibung der künstlichen Sequenz: Oligonucleotide
<220>
   <221> misc_feature
   <222> (1)..(21)
<400> 24
   ggagaacatc atggtcgaaa g 21
<210> 25
   <211> 22
   <212> DNA
   <213> Künstliche Sequenz
<220>
   <223> Beschreibung der künstlichen Sequenz: Oligonucleotide
<220>
   <221> misc_feature
   <222> (1)..(22)
<400> 25
   cccgagaaca tcatggtcga ag 22
<210> 26
   <211> 20
   <212> DNA
   <213> Künstliche Sequenz
<220>
   <223> Beschreibung der künstlichen Sequenz: Oligonucleotide
<220>
   <221> misc_feature
   <222> (1)..(20)
<400> 26
   ggggaaagcc cggcaagggg 20
<210> 27
   <211> 20
   <212> DNA
   <213> Künstliche Sequenz
<220>
   <223> Beschreibung der künstlichen Sequenz: Oligonucleotide
<220>
   <221> misc_feature
   <222> (1)..(20)
<400> 27
   cacccgcctt ggcctcccac 20
<210> 28
   <211> 18
   <212> DNA
   <213> Künstliche Sequenz
<220>
   <223> Beschreibung der künstlichen Sequenz: Oligonucleotide
<220>
   <221> misc_feature
   <222> (1)..(18)
<400> 28
   gggactccgg cgcagcgc 18
<210> 29
   <211> 20
   <212> DNA
   <213> Künstliche Sequenz
<220>
   <223> Beschreibung der künstlichen Sequenz: Oligonucleotide
<220>
   <221> misc_feature
   <222> (1)..(20)
<400> 29
   ggcaaacttt cttttcctcc 20
<210> 30
   <211> 19
   <212> DNA
   <213> Künstliche Sequenz
<220>
   <223> Beschreibung der künstlichen Sequenz: Oligonucleotide
<220>
   <221> misc_feature
   <222> (1)..(19)
<400> 30
   gggaaggagg aggatgagg 19
<210> 31
   <211> 21
   <212> DNA
   <213> Künstliche Sequenz
<220>
   <223> Beschreibung der künstlichen Sequenz: Oligonucleotide
<220>
   <221> misc_feature
   <222> (1)..(21)
<400> 31
   ggcagtcatc cagcttcgga g 21
<210> 32
   <211> 18
   <212> DNA
   <213> Künstliche Sequenz
<220>
   <223> Beschreibung der künstlichen Sequenz: Oligonucleotide
<220>
   <221> misc_feature
   <222> (1)..(18)
<400> 32
   tctcccagcg tgcgccat 18
<210> 33
   <211> 19
   <212> DNA
   <213> Künstliche Sequenz
<220>
   <223> Beschreibung der künstlichen Sequenz: Oligonucleotide
<220>
   <221> misc_feature
   <222> (1)..(19)
<400> 33
   gcgctgatag acatccatg 19
<210> 34
   <211> 12
   <212> DNA
   <213> Künstliche Sequenz
<220>
   <223> Beschreibung der künstlichen Sequenz: Oligonucleotide
<220>
   <221> misc_feature
   <222> (1)..(12)
<400> 34
   ggaggcccga cc 12
<210> 35
   <211> 12
   <212> DNA
   <213> Künstliche Sequenz
<220>
   <223> Beschreibung der künstlichen Sequenz: Oligonucleotide
<220>
   <221> misc_feature
   <222> (1)..(12)
<400> 35
   ggtttcggag gc 12
<210> 36
   <211> 12
   <212> DNA
   <213> Künstliche Sequenz
<220>
   <223> Beschreibung der künstlichen Sequenz: Oligonucleotide
<220>
   <221> misc_feature
   <222> (1)..(12)
<400> 36
   tggtggaggt ag 12
<210> 37
   <211> 12
   <212> DNA
   <213> Künstliche Sequenz
<220>
   <223> Beschreibung der künstlichen Sequenz: Oligonucleotide
<220>
   <221> misc_feature
   <222> (1)..(12)
<400> 37
   gcatggtgga gg 12
<210> 38
   <211> 12
   <212> DNA
   <213> Künstliche Sequenz
<220>
   <223> Beschreibung der künstlichen Sequenz: Oligonucleotide
<220>
   <221> misc_feature
   <222> (1)..(12)
<400> 38
   ttggcatggt gg 12
<210> 39
   <211> 12
   <212> DNA
   <213> Künstliche Sequenz
<220>
   <223> Beschreibung der künstlichen Sequenz: Oligonucleotide
<220>
   <221> misc_feature
   <222> (1)..(12)
<400> 39
   gcctgggacc ac 12
<210> 40
   <211> 12
   <212> DNA
   <213> Künstliche Sequenz
<220>
   <223> Beschreibung der künstlichen Sequenz: Oligonucleotide
<220>
   <221> misc_feature
   <222> (1)..(12)
<400> 40
   cagcctggga cc 12
<210> 41
   <211> 12
   <212> DNA
   <213> Künstliche Sequenz
<220>
   <223> Beschreibung der künstlichen Sequenz: Oligonucleotide
<220>
   <221> misc_feature
   <222> (1)..(12)
<400> 41
   tgcagcctgg ga 12
<210> 42
   <211> 12
   <212> DNA
   <213> Künstliche Sequenz
<220>
   <223> Beschreibung der künstlichen Sequenz: Oligonucleotide
<220>
   <221> misc_feature
   <222> (1)..(12)
<400> 42
   gtgcagcctg gg 12
<210> 43
   <211> 12
   <212> DNA
   <213> Künstliche Sequenz
<220>
   <223> Beschreibung der künstlichen Sequenz: Oligonucleotide
<220>
   <221> misc_feature
   <222> (1)..(12)
<400> 43
   ggtgcagcct gg 12
<210> 44
   <211> 12
   <212> DNA
   <213> Künstliche Sequenz
<220>
   <223> Beschreibung der künstlichen Sequenz: Oligonucleotide
<220>
   <221> misc_feature
   <222> (1)..(12)
<400> 44
   atgggtgcag cc 12
<210> 45
   <211> 12
   <212> DNA
   <213> Künstliche Sequenz
<220>
   <223> Beschreibung der künstlichen Sequenz: Oligonucleotide
<220>
   <221> misc_feature
   <222> (1)..(12)
<400> 45
   ggcttgaaga tg 12
<210> 46
   <211> 12
   <212> DNA
   <213> Künstliche Sequenz
<220>
   <223> Beschreibung der künstlichen Sequenz: Oligonucleotide
<220>
   <221> misc_feature
   <222> (1)..(12)
<400> 46
   gcagcccccg ca 12
<210> 47
   <211> 12
   <212> DNA
   <213> Künstliche Sequenz
<220>
   <223> Beschreibung der künstlichen Sequenz: Oligonucleotide
<220>
   <221> misc_feature
   <222> (1)..(12)
<400> 47
   gcagcagccc cc
<210> 48
   <211> 20
   <212> DNA
   <213> Künstliche Sequenz
<220>
   <223> Beschreibung der künstlichen Sequenz: Oligonucleotide
<220>
   <221> misc_feature
   <222> (1)..(20)
<400> 48
   tcccgcctgt gacatgcatt 20
<210> 49
   <211> 20
   <212> DNA
   <213> Künstliche Sequenz
<220>
   <223> Beschreibung der künstlichen Sequenz: Oligonucleotide
<220>
   <221> misc_feature
   <222> (1)..(20)
<400> 49
   gttctcgctg gtgagtttca 20
<210> 50
   <211> 18
   <212> DNA
   <213> Künstliche Sequenz
<220>
   <223> Beschreibung der künstlichen Sequenz: Oligonucleotide
<220>
   <221> misc_feature
   <222> (1)..(18)
<400> 50
   gcgtgcctcc tcactggc 18
<210> 51
   <211> 18
   <212> DNA
   <213> Künstliche Sequenz
<220>
   <223> Beschreibung der künstlichen Sequenz: Oligonucleotide
<220>
   <221> misc_feature
   <222> (1)..(18)
<400> 51
   gcagtaagca tccatatc 18
<210> 52
   <211> 20
   <212> DNA
   <213> Künstliche Sequenz
<220>
   <223> Beschreibung der künstlichen Sequenz: Oligonucleotide
<220>
   <221> misc_feature
   <222> (1)..(20)
<400> 52
   gcccaagctg gcatccgtca 20
<210> 53
   <211> 20
   <212> DNA
   <213> Künstliche Sequenz
<220>
   <223> Beschreibung der künstlichen Sequenz: Oligonucleotide
<220>
   <221> misc_feature
   <222> (1)..(20)
<400> 53
   cccccaccac ttcccctctc 20
<210> 54
   <211> 20
   <212> DNA
   <213> Künstliche Sequenz
<220>
   <223> Beschreibung der künstlichen Sequenz: Oligonucleotide
<220>
   <221> misc_feature
   <222> (1)..(20)
<400> 54
   ctcccccacc acttcccctc 20
<210> 55
   <211> 19
   <212> DNA
   <213> Künstliche Sequenz
<220>
   <223> Beschreibung der künstlichen Sequenz: Oligonucleotide
<220>
   <221> misc_feature
   <222> (1)..(19)
<400> 55
   gctgggagcc atagcgagg 19
<210> 56
   <211> 21
   <212> DNA
   <213> Künstliche Sequenz
<220>
   <223> Beschreibung der künstlichen Sequenz: Oligonucleotide
<220>
   <221> misc_feature
   <222> (1)..(21)
<400> 56
   actgctgcct cttgtctcag g 21
<210> 57
   <211> 22
   <212> DNA
   <213> Künstliche Sequenz
<220>
   <223> Beschreibung der künstlichen Sequenz: Oligonucleotide
<220>
   <221> misc_feature
   <222> (1)..(22)
<400> 57
   caatcaatga cttcaagagt tc 22
<210> 58
   <211> 18
   <212> DNA
   <213> Künstliche Sequenz
<220>
   <223> Beschreibung der künstlichen Sequenz: Oligonucleotide
<220>
   <221> misc_feature
   <222> (1)..(18)
<400> 58
   gcggcggaaa agccatcg 18
<210> 59
   <211> 18
   <212> DNA
   <213> Künstliche Sequenz
<220>
   <223> Beschreibung der künstlichen Sequenz: Oligonucleotide
<220>
   <221> misc_feature
   <222> (1)..(18)
<400> 59
   gtgtcggggt ctccgggc 18
<210> 60
   <211> 15
   <212> DNA
   <213> Kunstliche Sequenz
<220>
   <223> Beschreibung der künstlichen Sequenz: Oligonucleotide
<220>
   <221> misc_feature
   <222> (1)..(15)
<400> 60
   cacgttgagg ggcat 15
<210> 61
   <211> 18
   <212> DNA
   <213> Künstliche Sequenz
<220>
   <223> Beschreibung der künstlichen Sequenz: Oligonucleotide
<220>
   <221> misc_feature
   <222> (1)..(18)
<400> 61
   gtcttccata gttactca 18
<210> 62
   <211> 18
   <212> DNA
   <213> Künstliche Sequenz
<220>
   <223> Beschreibung der künstlichen Sequenz: Oligonucleotide
<220>
   <221> misc_feature
   <222> (1)..(18)
<400> 62
   gatcaggcgt gcctcaaa 18
<210> 63
   <211> 21
   <212> DNA
   <213> Künstliche Sequenz
<220>
   <223> Beschreibung der künstlichen Sequenz: Oligonucleotide
<220>
   <221> misc_feature
   <222> (1)..(21)
<400> 63
   gatggagggc ggcatggcgg g 21

## Beschreibung

Gegenstand der vorliegenden Erfindung ist die Verwendung von Arylresten der Formeln F1 und F3 bis F11, die an ein Polynukleotid, Oligonukleotid oder Mononukleotid gebunden sind (Konjugat), zum Transport dieses Moleküls über eine biologische Membran in vitro, aber auch zur Herstellung eines Arzneimittels zur Prävention und/oder Behandlung von Krankheiten, die mit der Expression bzw. einer Überexpression bestimmter Gene einhergehen, und/oder eines Diagnostikums für die Diagnose bzw. Früherkennung solcher Krankheiten. Gegenstand der vorliegenden Erfindung ist auch ein Verfahren zum Transport eines Moleküls in eine Zelle in vitro, wobei ein Arylrest der Formeln F1 und F3 bis F11 mit der an ein zu transportierendes Molekül ausgewählt aus einem Polynukleotid, Oligonukleotid oder Mononukleotid gebunden ist mit der Zelle inkubiert wird, woraufhin das Konjugat in die Zelle transportiert wird ohne daß der Aryl-Rest abgespalten wird.

Ein limitierender Faktor für die therapeutische Nutzung von Molekülen, deren Angriffspunkt sich innerhalb der Zelle befindet, ist oft deren unzulängliche zelluläre Aufnahme und ungünstige intrazelluläre Verteilung. Typische Beispiele sind Makromoleküle wie Nukleinsäuren, die in sequenzspezifischer Weise an die zelluläre DNA oder RNA binden und dadurch eine Inhibition der Genexpression bewirken. Antisense Oligonukleotide sind kurze einzelsträngige Nukleinsäuren, die über Watson-Crick Basenpaaren an eine komplementäre mRNA binden, deren Übersetzung in das entsprechende Protein gehemmt werden soll. Triplex-bildende Oligonukleotide binden über sogenannte "Hoogsteen-Basenpaarung" in die große Furche der DNA-Doppelhelix unter Ausbildung einer Tripelhelix, wodurch die Transkription der Gene sequenzspezifisch gehemmt wird. Andere intrazellulär wirkende Oligonukleotide sind beispielsweise die sogenannten "Decoy"-Oligonukleotide, die die Bindungsregionen für Transkriptionsfaktoren nachahmen. Durch die Behandlung mit Decoy-Oligonukleotiden lassen sich bestimmte Transkriptionsfaktoren sequenzspezifisch abfangen und dadurch eine Aktivierung der Transkription verhindern. Eine weitere Gruppe von intrazellulär wirkenden Oligonukleotiden, die Chimeraplasten, werden zur gezielten Genkorrektur herangezogen (Cole-Strauss et I., Science 273 (1996) 1386-1389). Auch für diese Genkorrektur ist die Aufnahme des Chimeraplast-Oligonukleotids in die Zelle essentiell. Beispiele für weitere intrazellulär wirkende Nukleinsäuren sind solche, welche mit zellulären Enzymen wechselwirken, insbesondere mit Telomerasen (Norton et al. Nat. Biotechn. (1996) 14, 615). Eine weitere Klasse von

Nukleinsäuren, bevorzugt doppelsträngige DNA, können für bestimmte Proteine kodieren, die intrazellulär exprimiert werden im Sinne einer Gentherapie.

Beispielsweise ist die Aufnahme eines Oligonukleotids in vitro in eine Zelle, z.B. durch einfache Zugabe des Oligonukleotids ins Zellkultur Medium ein relativ ineffizienter Prozess, da nur ein kleiner Teil des zugesetzten Oligonukleotids tatsächlich in die Zelle aufgenommen wird. Der Aufnahmeprozess dauert viele Stunden und meist wird erst nach 8 bis 16 Stunden eine Plateauphase erreicht. Man nimmt an, daß die Oligonukleotide in einem endozytoseartigen Prozess aufgenommen werden. Ein generelles Problem der Aufnahme über Endozytose besteht aber darin, daß ein Großteil der Oligonukleotide nicht frei im Zytoplasma, sondern in bestimmten Zellstrukturen, den Lysosomen und Endosomen, eingeschlossen vorliegt. Diese punktförmige Verteilung ist im Falle fluoreszenzmarkierter Oligonukleotide auch tatsächlich fluoreszenzmikroskopisch zu beobachten. Durch diese vesikuläre Lokalisierung ist die Konzentration an freiem Oligonukleotid, das tatsächlich für die Hybridisierung an die mRNA zur Verfügung steht, stark herabgesetzt. Außerdem nimmt, je nach Zelltyp und vorliegenden Bedingungen, nur eine bestimmte Fraktion der Zellen das Oligonukleotid überhaupt auf. Daher werden zur effizienten Anwendung von Antisense Oligonukleotiden im allgemeinen Mischungen mit Penetrationsverstärkern wie beispielsweise kationische Lipide ( Bennett et al. Mol. Pharmacol. 41 (1992) 1023) zur Anwendung gebracht.

WO 95/06659 beschreibt aminoalkyl-derivatisierte Oligonukleotide mit verbesserter zellulärer Membranpermeabilität. EP 0 962 497 beschreibt Rhodaminderivate und deren Verwendung in diagnostischen Systemen und Whittemore, N. A. et al. (1999) Bioconjugate Chem., 10, 261-270 beschreiben Anthraquinon-Oligodeoxynukleotid-Konjugate.

Eine Aufgabe der vorliegenden Erfindung bestand darin, die zelluläre Aufnahme von Molekülen, insbesondere von Makromolekülen wie beispielsweise Oligonukleotiden, zu verbessern.

Die Untersuchung der zellulären Aufnahme von Oligonukleotiden erfolgt im allgemeinen entweder mit Hilfe radioaktiv markierter oder fluoreszenzmarkierter Oligonukleotide. Die Fluoreszenzmarkierung eines Oligonukleotids erfolgt beispielsweise durch Umsetzung der Aminofunktion eines Oligonukleotids mit Fluoresceinisothiocyanat (FITC). Zum Beispiel kann das Fluorescein an das 3'-Ende eines Oligonukleotids über einen kommerziell erhältlichen Fluorescein-derivatisierten

Festphasenträger oder an das 5'-Ende über ein kommerziell erhältliches Fluorescein-Phosphitylierungsreagenz eingeführt werden. In allen Fällen liegt das an das Oligonukleotid gebundene Fluorescein aufgrund der Carbonsäurefunktion als negativ geladenes Strukturelement vor, das stark fluoreszierend ist.

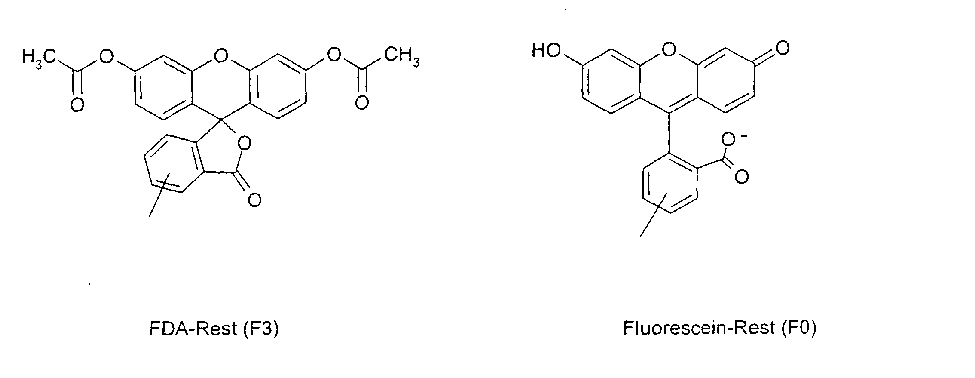

Im Gegensatz zu Fluorescein ist Fluoresceindiacetat (FDA) ein neutraler Vitalfarbstoff, der erst nach Spaltung der beiden Estergruppen und Öffnung des Lacton-Rings in das fluoreszierende Fluorescein übergeht, der aber in Form des Lactons noch keine Fluoreszenz aufzeigt.

Es ist bekannt, daß FDA (nachfolgend auch als "F3" bezeichnet) als neutrales nichtfluoreszierendes Molekül von lebenden Zellen durch passive Diffusion aufgenommen und intrazellulär von Esterasen in das fluoreszierende Fluorescein gespalten wird (Breeuwer et al. Appl. Environ. Microbiol. (1995) 61, 1614; Maeda et al. Cell Struct. Funct. (1982) 7, 177). Bisher wurden nur FDA-Derivate, die eine Amin-reaktive Gruppe wie z.B. Isothiocyanat enthalten, beschrieben; diese FDA-Derivate werden zur Färbung von intrazellulären Proteinen oder Zellkomponenten eingesetzt. Darüber hinaus wurden bisher keine Konjugate von FDA mit anderen Molekülen beschrieben; entsprechend wurden auch FDA-markierte Oligonukleotide (Konjugate aus FDA und Oligonukleotid) bisher nicht beschrieben.

Im Zytoplasma wird FDA durch Esterasen gespalten; durch eine FDA-Markierung eines Oligonukleotids ist es deshalb möglich, den Anteil an "freiem" Oligonukleotid zu bestimmen, d.h. wieviel Oligonukleotid im Zytoplasma vorliegt - und für die Hybridisierung zur Verfügung steht - im Verhältnis zu dem Anteil an Oligonukleotid, der in Vesikeln vorliegt ("gefangenes" Oligonukleotid) - und deshalb nicht für die Hybridisierung zur Verfügung steht. Bedingt durch die insgesamt hohe Anzahl negativer Ladungen in einem Oligonukleotid und die Tatsache, daß sich FDA-markierte und Fluorescein-markierte Oligonukleotide (für den Fall, daß das Oligonukleotid das gleiche ist) nur durch eine Nettoladung unterscheiden, war zu erwarten, daß FDA-markierte und Fluorescein-markierte Oligonukleotide eine sehr ähnliche zelluläre Aufnahme und Verteilung zeigen würden.

Überraschend wurde jedoch gefunden, daß sich FDA-markierte und Fluorescein-markierte Oligonukleotide deutlich im Bezug auf ihre Aufnahme in Zellen unterscheiden und zwar sowohl im Hinblick auf die Dauer als auch die Effizienz der Aufnahme der Oligonukleotide und darüber hinaus auch im Hinblick auf die Lokalisation der aufgenommenen Oligonukleotide in der Zelle. Ein mit FDA markiertes Oligonukleotid wird sehr viel schneller von Zellen aufgenommen als das entsprechende Fluorescein-markierte Oligonukleotid. Während die Aufnahme radioaktiv-markierter und Fluorescein-markierter Oligonukleotide mehrere Stunden erfordert, konnten die FDA-markierten Oligonukleotide nach einfacher Inkubation, beispielsweise mit humanen Zellen, bereits nach fünf Minuten intrazellulär nachgewiesen werden. Überraschend war auch, daß die FDA-markierten Oligonukleotide in nahezu alle Zellen (>90% der Zellen) aufgenommen wurden, während die Aufnahmerate bei den bisher beschriebenen Methoden Oligonukleotide bzw. Polynukleotide in Zellen einzubringen im allgemeinen wesentlich niedriger ausfällt; häufig werden dort nur etwa 30 bis 60% der Zellen mit Oligonukleotiden beladen. Vorteilhaft ist auch die intrazelluläre Verteilung der FDA-markierten Oligonukleotide, die viel gleichmäßiger ist. Diese gleichmäßigere Verteilung deutet darauf hin, daß die Oligonukleotide nicht - wie oben beschrieben - zum großen Teil in Vesikeln (z.B. Endosomen, Lysosomen) eingeschlossen vorliegen, sondern, in der gesamten Zelle - d.h. im Zytosol und im Nucleus - verteilt sind; dies ist ein Indiz dafür, daß ein großer Anteil an "freiem" Oligonukleotid vorliegt. Nur diese "freien" Oligonukleotide stehen für die Bindung an das Target (Zielmolekül, Zielnukleinsäure) oder als Wirkstoff zur Verfügung. Von Vorteil ist auch, daß mit den FDA-markierten Oligonukleotiden keine Schädigung der Zellen beobachtet werden konnte; im Gegensatz dazu führt die Verwendung von lipokationischen Penetrationsverstärkern oft zu Schädigungen der Zellmembran. Bedingt durch diese unerwarteten Eigenschaften haben die FDA-markierten Oligonukleotide gegenüber den bisher beschriebenen Methoden Oligonukleotide bzw. Polynukleotide in Zellen einzubringen den entscheidenden Vorteil, daß sie effektiver in die Zellen eingebracht werden können und dort auch besser verfügbar sind. Dadurch bedingt weisen die FDA-markierten Oligonukleotide eine deutlich verbesserte biologische Wirksamkeit auf. Aufgrund der verbesserten biologischen Wirksamkeit muß weniger Oligonukleotid eingesetzt werden. Dadurch und aufgrund der Tatsache, daß ein FDA-markiertes Oligonukleotid effektiver - sowohl mengenmäßig als auch zeitlich - in eine Zelle aufgenommen wird, werden (toxische) Nebenwirkungen reduziert.

Überraschend wurde gefunden, daß die vorteilhaften Eigenschaften nicht auf FDA-markierte Oligonukleotide beschränkt sind, sondern praktisch jedes Molekül mit Hilfe einer FDA-Markierung - d.h. dadurch, das ein zu transportierendes Molekül an FDA gekoppelt bzw. konjugiert wird ("FDA-Konjugat") - effektiv in eine Zelle eingebracht bzw. über eine biologische Membran transportiert werden kann. Weiterhin wurde gefunden, daß dieses Prinzip nicht auf FDA-Konjugate beschränkt ist, sondern sich auf alle Arylester-Konjugate, die eine bestimmte chemische Struktur aufweisen, erstrecken. Die vorliegende Erfindung stellt somit ein neues Prinzip zum Transport von Molekülen über biologische Membranen bereit.

Bekannt sind bioreversible O-Acylaryl-Konjugate, die als Pro-Drugs von Oligonukleotiden vorgeschlagen wurden (lyer et al., Bioorganic & Med. Chem. Lett. 7 (1997) 871-876). Diese Verbindungen ähneln - für den Fall, daß der Aryl-Rest ein aromatischer 6-Ring ist - in ihrer chemischen Struktur den erfindungsgemäßen Konjugaten. Bei den bioreversiblen O-Acylaryl-Konjugaten bewirkt die Hydrolyse des Esters jedoch eine Destabilisierung der Bindung zwischen dem Aryl-Rest und dem Phosphotriester des Oligonukleotids, so daß das bioreversible O-Acylaryl-Konjugate in seine Bestandteile, d.h. in das freie Oligonukleotid und die O-Acylaryl-Rest, gespalten. Dieses Pro-Drug-Konzept dient dazu, die negative Ladung der Internukleotid-Phosphatbrücke zu maskieren und dadurch die Aufnahme des Oligonukleotids in die Zelle zu erleichtern. Im Gegensatz zu den erfindungsgemäßen Konjugaten wurde bei diesen Pro-Drugs aber keine beschleunigte Aufnahme der Oligonukleotide in die Zellen und auch keine veränderte intrazellulären Verteilung der Oligonukleotide festgestellt. Weiterhin wurde nicht über eine Aufnahme der Oligonukleotide in andere Organismen berichtet. Im Gegensatz dazu bleibt bei den erfindungsgemäßen Konjugate die kovalente Bindung zwischen dem Aryl-Rest und dem Oligonukleotid bei der Aufnahme in die Zelle erhalten; der Erhalt der kovalenten Bindung zwischen Aryl-Rest und Oligonukleotid kann leicht fluoreszenzmikroskopisch festgestellt werden, sofern die aromatische Einheit, wie beispielsweise bei FDA, erst nach Spaltung des Esters eine Fluoreszenz aufweist.

Gegenstand der vorliegenden Erfindung ist die Verwendung eines Arylrestes der Formeln F1 und F3 bis F11, mit 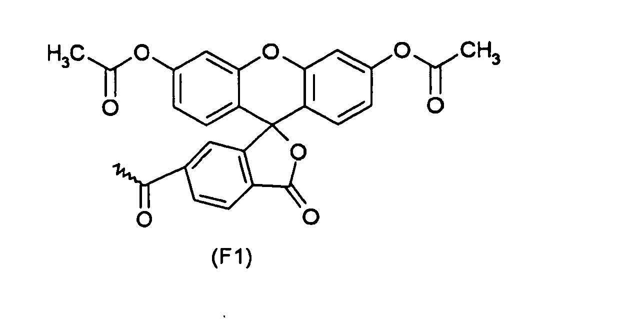 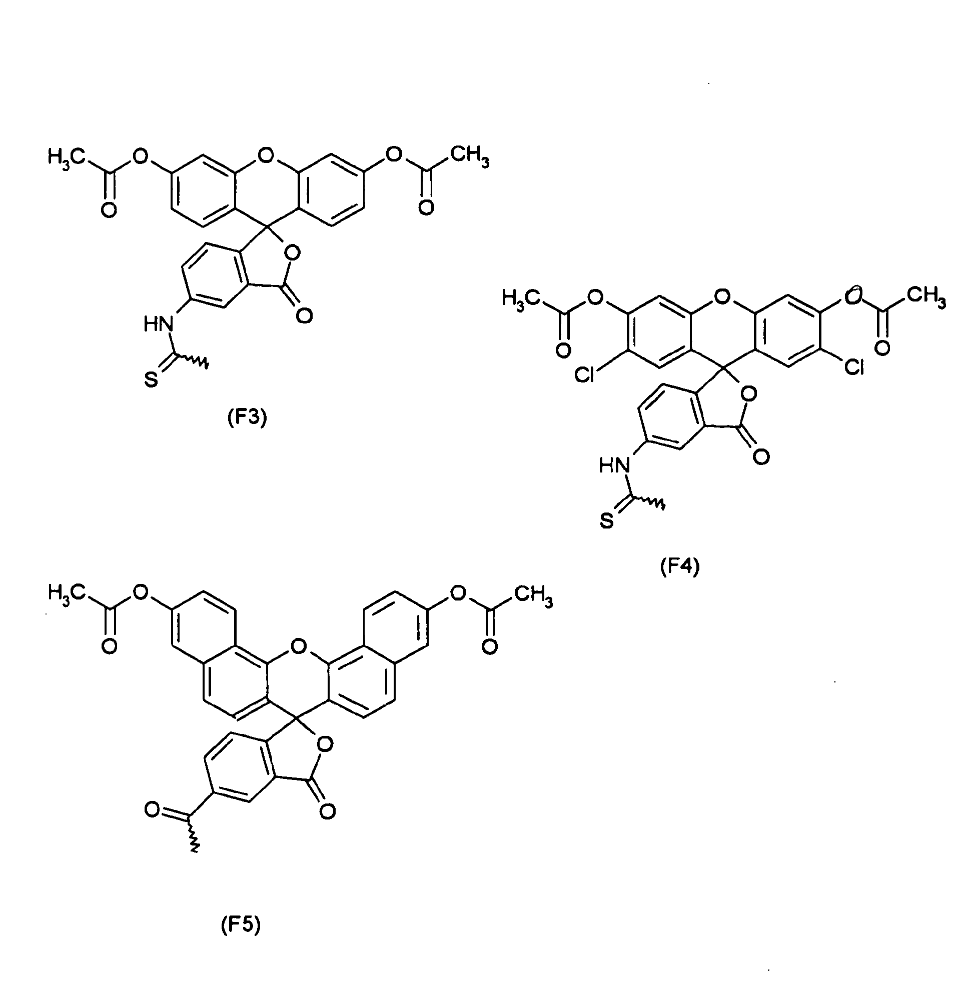 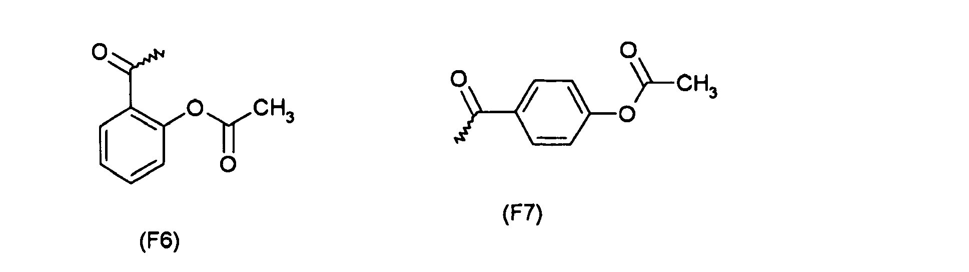 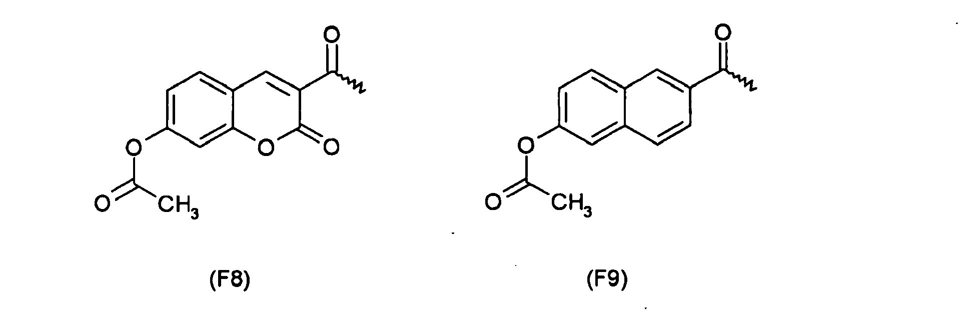 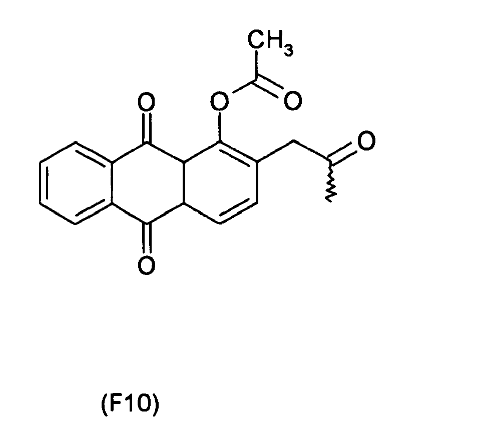 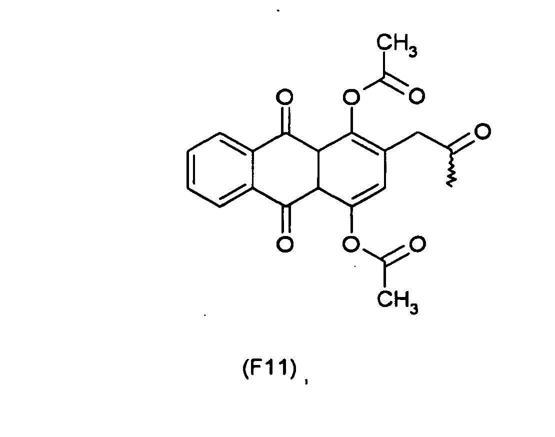 der an ein zu transportierendes Molekül ausgewählt aus einem Polynukleotid, Oligonukleotid oder Mononukleotid gebunden ist, zum Transport dieses Moleküls über eine biologische Membran in vitro.

Ein weiterer Gegenstand der vorliegenden Erfindung ist die Verwendung eines Arylrestes der Formeln F1 und F3 bis F11, mit 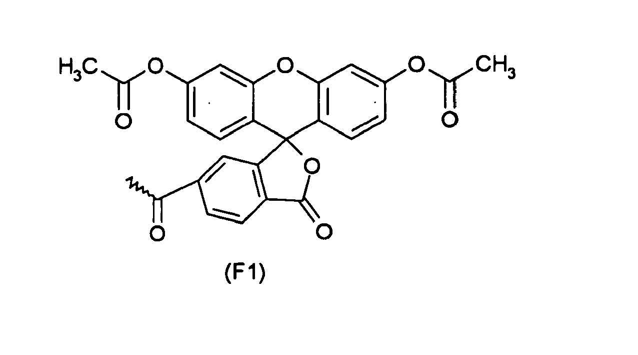 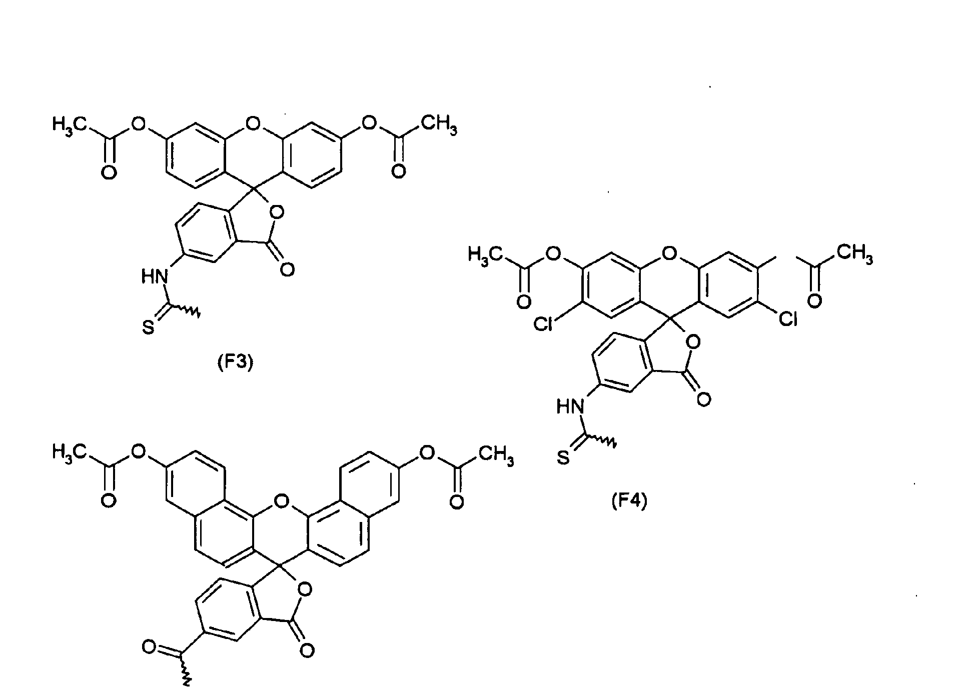 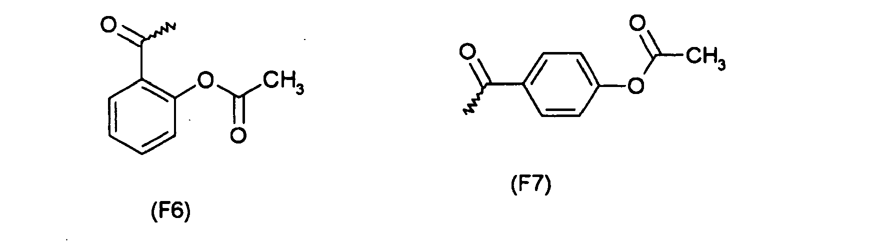 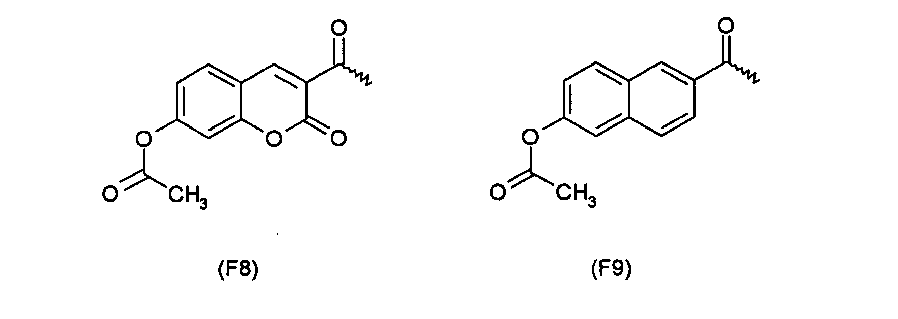 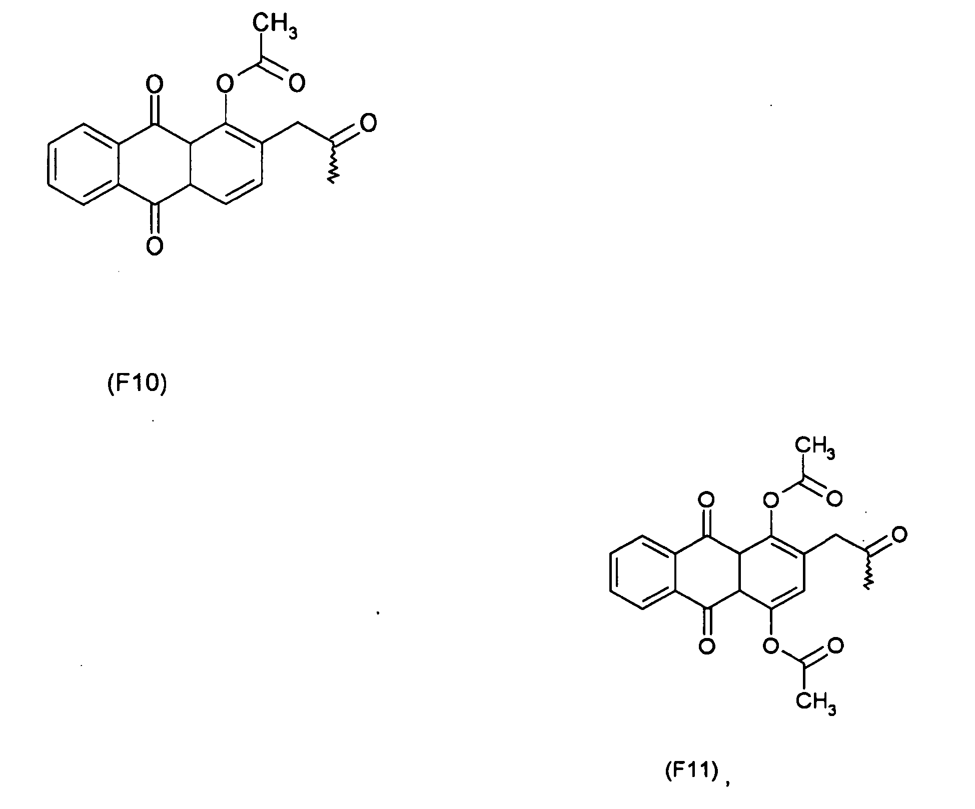 der an ein zu transportierendes Molekül ausgewählt aus einem Polynukleotid, Oligonukleotid oder Mononukleotid gebunden ist, zur Herstellung eines Arzneimittels zur Prävention und/oder Behandlung von Krankheiten, die mit der Expression bzw. einer Überexpression bestimmter Gene einhergehen, und/oder eines Diagnostikums für die Diagnose bzw. Früherkennung solcher Krankheiten.

In einer besonderen Ausführungsform ist das zu transportierende Molekül ein Oligonukleotid. Ein Oligonukleotid kann beispielsweise vollständig aus den Nukleotiden Adenosinphosphat, Guanosinphosphat, Inosinphosphat, Cytidinphosphat, Uridinphosphat und Thymidinphosphat aufgebaut sein. In anderen Ausführungsformen der Erfindung kann ein Oligonukleotid gegebenenfalls ein oder mehrere Modifikationen, beispielsweise chemische Modifikationen, enthalten. Ein Oligonukleotid kann mehrere gleiche und/oder verschiedene Modifikationen aufweisen.

Beispiele für chemische Modifikationen sind dem Fachmann bekannt und beispielsweise in E. Uhlmann and A. Peyman, Chemical Reviews 90 (1990) 543 und "Protocols for Oligonukleotides and Analogs" Synthesis and Properties & Synthesis and Analytical Techniques, S. Agrawal, Ed, Humana Press, Totowa, USA 1993 und J. Hunziker und C. Leumann 'Nucleic Acid Analogs: Sythesis and Properties' in Modern Synthetic Methods (Ed. Beat Ernst und C. Leumann) Verlag Helvetica Chimica Acata, Basel, S 331-417, beschrieben.

Die chemische Modifikation eines Oligonukleotids kann beispielsweise
a) den vollständigen oder teilweisen Ersatz der Phosphorsäurediesterbrücken, beispielsweise durch Phosphorothioat-, Phoshorodithioat-, NR¹R^{1'}-Phosphoramidat-, Boranophosphat-, Phosphat-(C₁-C₂₁)-O-Alkylester, Phosphat-[(C₆-C₁₂)Aryl-(C₁-C₂₁)-O-Alkyl]ester, (C₁-C₈)Alkylphosphonat- und/oder (C₆-C₁₂)-Arylphosphonat-Brücken, wobei
   R¹ und R^{1'} unabhängig voneinander für Wasserstoff, (C₁-C₁₈)-Alkyl, (C₆-C₂₀)-Aryl, (C₆-C₁₄)-Aryl-(C₁-C₈)-alkyl, bevorzugt für Wasserstoff, (C₁-C₈)-Alkyl und/oder Methoxyethyl, besonders bevorzugt für Wasserstoff, (C₁-C₄)-Alkyl und/oder Methoxyethyl stehen
   oder
   R¹ und R^{1'} zusammen mit dem sie tragenden Stickstoffatom einen 5-6-gliedrigen heterocyclischen Ring bilden, der zusätzlich ein weiteres Heteroatom aus der Reihe O, S, N enthalten kann;
b) den vollständigen oder teilweisen Ersatz der 3'- und/oder 5'-Phosphorsäurediesterbrücken durch "Dephospho"-Brücken (beschrieben beispielsweise in Uhlmann, E. und Peyman, A. in "Methods in Molecular Biology", Vol. 20, "Protocols for Oligonukleotides and Analogs", S. Agrawal, Ed., Humana Press, Totowa 1993, Chapter 16, 355ff), beispielsweise durch Formacetal, 3'-Thioformacetal, Methylhydroxylamin, Oxim, Methylendimethylhydrazo, Dimethylensulfon und/oder Silylgruppen;
c) den vollständigen oder teilweisen Ersatz des Zuckerphosphat-Rückgrats, beispielsweise durch "Morpholino"-Oligomere (beispielweise in E. P. Stirchak et al., Nucleic Acids Res. 17 (1989) 6129 und in J. Summerton and D. Weller, Antisense and Nucleic Acid Drug Dev. 7 (1997) 187-195 beschrieben) und/oder durch Polyamid Nukleinsäuren ("PNAs") (beispielsweise beschrieben in P. E. Nielsen et al, Bioconj. Chem. 5 (1994) 3) und/oder Phosphomonosäureester Nukleinsäuren ("PHONAs") (beschrieben beispielsweise in Peyman et al., Angew. Chem. Int. Ed. Engl. 35 (1996) 2632-2638);
d) den vollständigen und/oder teilweisen Ersatz der β-D-2'-Desoxyriboseeinheiten, beispielsweise durch α-D-2'-Desoxyribose, L-2'-Desoxyribose, 2'-F-2'-Desoxyribose, 2'-O-(C₁-C₆)Alkyl-Ribose, 2'-O-(C₂-C₆)Alkenyl-Ribose,2'-[O-(C₁-C₆)Alkyl-O-(C₁-C₆)Alkyl]-Ribose, 2'-NH₂-2'-desoxyribose, β-D-Xylofuranose, α-Arabinofuranose, 2,4-Dideoxy-β-D-erythro-hexo-pyranose, conformativ eingeschränkte Zuckeranaloga wie LNA (Locked nucleic acids; Singh et al., Chem. Commun. 4 (1998) 455; Singh et al. Chem. Commun. 12 (1998) 1247) und carbocyclische (beschreiben beispielsweise in Froehler, J.Am.Chem.Soc. 114 (1992) 8320) und/oder offenkettige Zuckeranaloga (beschrieben beispielsweise in Vandendriessche et al., Tetrahedron 49 (1993) 7223) und/oder Bicyclozuckeranaloga (beschrieben beispielsweise in M. Tarkov et al., Helv. Chim. Acta 76 (1993) 481);
e) die Modifikation beziehungsweise den vollständigen oder teilweisen Ersatz der natürlichen Nucleosid-Basen, beispielsweise durch 5-(Hydroxymethyl)uracil, 5-Aminouracil, Pseudouracil, Pseudoisocytosin, Dihydrouracil, 5-(C₁-C₆)-Alkyl-uracil, 5-(C₂-C₆)-Alkenyl-uracil, 5-(C₂-C₆)-Alkinyl-uracil, 5-(C₁-C₆)-Alkyl-cytosin, 5-(C₂-C₆)-Alkenyl-cytosin, 5-(C₂-C₆)-Alkinyl-cytosin, 5-Fluoruracil, 5-Fluorcytosin, 5-Chloruracil, 5-Chlorcytosin, 5-Bromuracil, 5-Bromcytosin oder 7-Deaza-7-substituierte Purine beinhalten.

Die chemische Modifikation eines Oligonukleotids umfaßt weiterhin die Bindung eines Oligonukleotids an ein oder mehrere weitere Moleküle, die besondere Eigenschaften des Oligonukleotids, z.B. Nukleasestablilität, Affinität zur Target-Sequenz und Pharmakokinetik günstig beeinflußen, z.B. bei der Hybridisierung des modifizierten Oligonukleotids an die Target-Sequenz diese unter Bindung und/oder Quervernetzung angreifen. Beispiele dafür solche weiteren Moleküle sind Poly-Lysin, Interkalatoren wie Pyren, Acridin, Phenazin und Phenanthridin, fluoreszierende Verbindungen wie Fluorescein, Cross-Linker wie Psoralen und Azidoproflavin, lipophile Moleküle wie (C₁₂-C₂₀)-Alkylgruppen, vorzugsweise (C₁₂-C₂₀)-Alkylgruppen, Lipide wie 1,2-Di-hexadecyl-rac-glycerin, Steroide wie Cholesterin oder Testosteron, Vitamine wie Vitamin E, Poly- bzw. Oligo-ethylengylcol, (C₁₂-C₁₈)-Alkyl-Phosphatdiestern, vorzugsweise(C₁₄-C₁₈)-Alkyl-Phosphatdiestern und -O-CH₂-CH(OH)-O-(C₁₂-C₁₈)-Alkylgruppen, vorzugsweise -O-CH₂-CH(OH)-O-(C₁₂-C₁₆)-Alkylgruppen. Diese weiteren Moleküle können am 5'- und/oder am 3'-Ende und/oder innerhalb der Sequenz, z.B. an eine Nukleobase konjugiert sein. Die Verfahren zur Herstellung derart modifizierter Oligonukleotide sind dem Fachmann bekannt und z.B. in Uhlmann, E. & Peyman, A., Chem. Rev. 90 (1990) 543 und/oder M. Manoharan in "Antisense Research and Applications", Crooke and Lebleu, Eds., CRC Press, Boca Raton, 1993, Chapter 17, S.303ff. und/oder EP-A 0 552 766 beschrieben.

In weiteren, speziellen Ausführungsformen der Erfindung kann das Oligonukleotid am 3' und/oder am 5'-Ende 3'-3'- und/oder 5'-5'-Inversionen aufweisen. Diese Art der chemischen Modifikation ist dem Fachmann bekannt und beispielsweise in M. Koga et al., J. Org. Chem. 56 (1991) 3757 beschrieben.

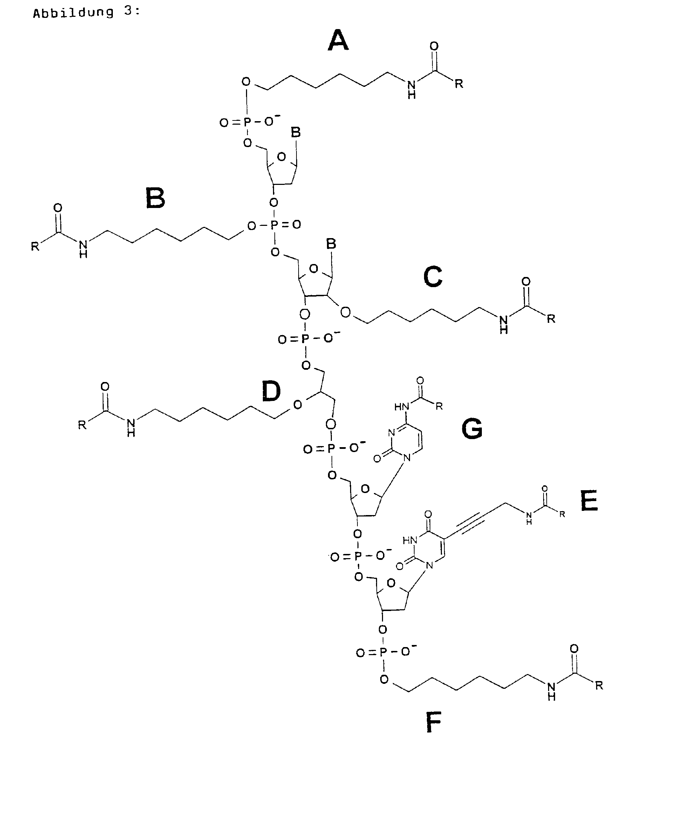

Bei einem Konjugat, daß aus einem oder mehreren Oligonukleotiden und einem oder mehreren Aryl-Resten der Formeln F1 und F3 bis F11 besteht, kann die Anbindung (Konjugation) von Aryl-Resten an ein Oligonukleotid beispielsweise am 5'-Ende (A), am 3'-Ende (F), an die heterozyklische Base (E und G), an den Zucker (C) oder an die Internukleosidbrücke (B) des Oligonukleotids erfolgen. Die Anbindung kann zum Beispiel aber auch über nichtnukleotidische Bausteine, z.B. Fall (D) erfolgen. Diese Beispiele sind in Abbildung 3 dargestellt.

Die genannten Modifikationen können entsprechend natürlich auch auf länger Polynukleotide und soweit geeignet Mono- bzw. Dinukleotide bzw. -nukleoside angewendet werden.

Die Oligonukleotide haben beispielsweise eine Länge von 8 bis 50 Nukleotiden, vorzugsweise 10-20 Nukleotide. Geeignet sind aber auch Oligonukleotide längere Oligo- bzw. Polyonukleotide, beispielsweise mit einer Länge von 50 bis 10000 Nukleotiden, vorzugsweise 100 bis 1000 Nukleotiden, die gegebenenfalls auch doppelsträngig vorliegen können.

Die Oligonukleotide können jede beliebige Sequenz aufweisen. Abhängig von dem ausgewählten Target, d.h. falls das Target eine Nukleinsäure ist, in Anhängigkeit von dessen Sequenz oder falls das Target ein Protein ist, in Abhängigkeit von der Nukleinsäuresequenz, die für dieses Target-Protein kodiert, wird die Sequenz des Oligonukloeitds ausgewählt bzw. designed. Ist beispielsweise das Target ein Virus, z.B. CMV, HIV, HSV-1, HSV-2, Influenza, VSV, Hepatitis B oder Papilloma Virus, dann kann das Oligonukleotid z.B. eine der folgenden Sequenzen haben:
a) gegen CMV

| | |
|---|---|
| SEQ ID NO. 12 | 5'-GCGTTTGCTCTTCTTCTTGCG |

b) gegen HIV, z. B.

| | | |
|---|---|---|
| SEQ ID NO. 13 | 5'-ACACCCAATTCTGAAAATGG-3' | oder |
| SEQ ID NO. 14 | 5'-AGGTCCCTGTTCGGGCGCCA-3' | oder |

c) gegen HSV-1, z.B.

| | |
|---|---|
| SEQ ID NO. 15 | 5'-GCGGGGCTCCATGGGGGTCG-3' |

Das Target kann z.B. ein Protein sein, das an der Krebsentstehung beteiligt bzw. für das Krebswachstum verantwortlich ist. Beispiele für solche Targets sind:
1) Nucleare Onkoproteine wie beispielsweise c-myc, N-myc, c-myb, c-fos, c-fos/jun, PCNA, p120;
2) Cytoplasmische/Membran-assoziierte Onkoproteine wie beispielsweise EJ-ras, c-Ha-ras, N-ras, rrg, bcl-2, cdc-2, c-raf-1, c-mos, c-src, c-abl, c-ets;
3) Zelluläre Rezeptoren wie beispielsweise EGF-Rezeptor, Her-2, c-erbA, VEGF-Rezeptor (KDR-1), Retinoid-Rezeptoren, Protein-Kinase regulatorische Untereinheit, c-fms, Tie-2, c-raf-1 Kinase, PKC-alpha, Protein Kinase A (R1 alpha);
4) Cytokine, Wachstumsfaktoren, Extrazelluläre Matrix wie beispielsweise CSF-1, IL-6, IL-1a, IL-1b, IL-2, IL-4, IL-6, IL-8, bFGF, VEGF, Myeloblastin, Fibronectin.

Oligonukleotide, die gegen solche Targets gerichtet sind können beispielsweise folgende Basen-Sequenz haben:
a) gegen c-Ha-ras, z. B.

| | |
|---|---|
| SEQ ID NO. 16 | 5'-CAGCTGCAACCCAGC-3' oder |
| SEQ ID NO. 17 | 5'-TATTCCGTCAT-3' oder |
| SEQ ID NO. 18 | 5'-TTCCGTCATCGCTCCTCAGGGG-3' |

b) bFGF, z.B.

| | |
|---|---|
| SEQ ID NO. 19 | 5'-GGCTGCCATGGTCCC-3' |

c) c-myc, z.B.

| | |
|---|---|
| SEQ ID NO. 20 | 5'-GGCTGCTGGAGCGGGGCACAC-3' |
| SEQ ID NO. 21 | 5'-AACGTTGAGGGGCAT-3' |

d) c-myb, z.B.

| | |
|---|---|
| SEQ ID NO. 22 | 5'-GTGCCGGGGTCTTCGGGC-3' |

e) c-fos, z.B.

| | |
|---|---|
| SEQ ID NO. 23 | 5'-CGAGAACATCATCGTGG-3' |
| SEQ ID NO. 24 | 5'-GGAGAACATCATGGTCGAAAG-3' |
| SEQ ID NO. 25 | 5'-CCCGAGAACATCATGGTCGAAG-3' |
| SEQ ID NO. 26 | 5'-GGGGAAAGCCCGGCAAGGGG-3' |

f) p120, z.B.

| | |
|---|---|
| SEQ ID NO. 27 | 5'-CACCCGCCTTGGCCTCCCAC-3' |

g) EGF-Rezeptor, z.B.

| | |
|---|---|
| SEQ ID NO. 28 | 5'-GGGACTCCGGCGCAGCGC-3' |
| SEQ ID NO. 29 | 5'-GGCAAACTTTCTTTTCCTCC-3' |

h) p53 Tumorsuppressor, z.B.

| | |
|---|---|
| SEQ ID NO. 30 | 5'-GGGAAGGAGGAGGATGAGG-3' |
| SEQ ID NO. 31 | 5'-GGCAGTCATCCAGCTTCGGAG-3' |

i) bcl-2

| | |
|---|---|
| SEQ ID NO. 32 | 5'-TCTCCCAGCGTGCGCCAT |

k) VEGF

| | |
|---|---|
| SEQ ID NO. 33 | 5'-GCGCTGATAGACATCCATG |
| SEQ ID NO. 34 | 3'- CCAGCCCGGAGG -5', 5'-GGAGGCCCGACC-3' |
| SEQ ID NO. 35 | 3'- CGGAGGCTTTGG -5', 5'-GGTTTCGGAGGC-3'; |
| SEQ ID NO. 36 | 3'- GATGGAGGTGGT -5', 5'-TGGTGGAGGTAG-3' |
| SEQ ID NO. 37 | 3'- GGAGGTGGTACG -5', 5'-GCATGGTGGAGG-3' |
| SEQ ID NO. 38 | 3'- GGTGGTACGGTT -5', 5'-TTGGCATGGTGG-3' |
| SEQ ID NO. 39 | 3'- CACCAGGGTCCG -5', 5'-GCCTGGGACCAC-3' |
| SEQ ID NO. 40 | 3'- CCAGGGTCCGAC -5', 5'-CAGCCTGGGACC-3' |
| SEQ ID NO. 41 | 3'- AGGGTCCGACGT -5', 5'-TGCAGCCTGGGA-3' |
| SEQ ID NO. 42 | 3'- GGGTCCGACGTG -5', 5'-GTGCAGCCTGGG-3' |
| SEQ ID NO. 43 | 3'- GGTCCGACGTGG -5', 5'-GGTGCAGCCTGG-3' |
| SEQ ID NO. 44 | 3'- CCGACGTGGGTA -5', 5'-ATGGGTGCAGCC-3' |
| SEQ ID NO. 45 | 3'- GTAGAAGTTCGG -5', 5'-GGCTTGAAGATG-3' |
| SEQ ID NO. 46 | 3'- ACGCCCCCGACG -5', 5'-GCAGCCCCCGCA-3' oder |
| SEQ ID NO. 47 | 3'- CCCCCGACGACG -5', 5'-GCAGCAGCCCCC-3' |

l) c-raf Kinase

| | |
|---|---|
| SEQ ID NO. 48 | 5'- TCCCGCCTGTGACATGCATT |

m) PKC-alpha

| | |
|---|---|
| SEQ ID NO. 49 | 5'-GTTCTCGCTGGTGAGTTTCA |

n) Protein Kinase A

| | |
|---|---|
| SEQ ID NO. 50 | 5'-GCGTGCCTCCTCACTGGC |

Ist das Target ein Integrin oder ein Zell-Zell-Adhäsionsrezeptor, wie z.B. VLA-4, VLA-2, ICAM, VCAM oder ELAM, dann kann das Oligonukleotid beispielsweise eine der folgenden Sequenzen haben:
a) VLA-4, z.B.

| | |
|---|---|
| SEQ ID NO. 51 | 5'-GCAGTAAGCATCCATATC-3' oder |

b) ICAM-1, z.B.

| | |
|---|---|
| SEQ ID NO. 52 | 5'-GCCCAAGCTGGCATCCGTCA |
| SEQ ID NO. 53 | 5'-CCCCCACCACTTCCCCTCTC-3' |
| SEQ ID NO. 54 | 5'-CTCCCCCACCACTTCCCCTC-3' |
| SEQ ID NO. 55 | 5'-GCTGGGAGCCATAGCGAGG-3' |

c) ELAM-1, z. B.

| | |
|---|---|
| SEQ ID NO. 56 | 5'-ACTGCTGCCTCTTGTCTCAGG-3' |
| SEQ ID NO. 57 | 5'-CAATCAATGACTTCAAGAGTTC-3' |

d) Integrin alpha(V)

| | |
|---|---|
| SEQ ID NO. 58 | 5'-GCGGCGGAAAAGCCATCG |

Ist das Target ein Protein, das für Proliferation oder Migration verantwortlich bzw. an diesen/diesem Prozess(en) beteiligt ist, wie beispielsweise:
1) Nucleare Transaktivator-Proteine und Cycline wie beispielsweise c-myc, c-myb, c-fos, c-fos/jun, Cycline und cdc2-Kinase;
2) Mitogene oder Wachstumsfaktoren wie beispielsweise PDGF, bFGF, VEGF, EGF, HB-EGF und TGF-β;
3) Zelluläre Rezeptoren wie beispielsweise bFGF-Rezeptor, EGF-Rezeptor und PDGF-Rezeptor;
dann kann das Oligonukleotid beispielsweise eine der folgenden Basen-Sequenzen haben:
a) c-myb

| | |
|---|---|
| SEQ ID NO. 59 | 5'-GTGTCGGGGTCTCCGGGC-3' |

b) c-myc

| | |
|---|---|
| SEQ ID NO. 60 | 5'-CACGTTGAGGGGCAT-3' |

c) cdc2-Kinase

| | |
|---|---|
| SEQ ID NO. 61 | 5'-GTCTTC CATAGTTACTCA-3' |

d) PCNA (proliferating cell nuclear antigen of rat)

| | |
|---|---|
| SEQ ID NO. 62 | 5'-GATCAGGCGTGCCTCAAA-3'. |

Ist das Target z.B. ein Adenosin-A1-Rezeptors, Adenosin-A3-Rezeptors, Bradikinin-Rezeptors oder IL-13, ist beispielsweise die Basen-Sequenz

| | |
|---|---|
| SEQ ID NO. 63 | 5'-GATGGAGGGCGGCATGGCGGG |

möglich.

Folgende Oligonukleotide (5'-->3') wurden hergestellt:

| | |
|---|---|
| ON1: 5'-d(G*C G A C*G C*C A T*G A C*G*G) | SEQ ID NO. 1 |
| ON2: 5'-d(C*G A C*G C*C A T*G*A*C) | SEQ ID NO. 2 |
| ON3: 5'-d(A*T*G A C*G G A A*T*T*C) | SEQ ID NO. 3 |
| ON4: 5'-d(TATTCCGTCAT) | SEQ ID NO. 4 |
| ON5: 5'-(dA)₂₀ | SEQ ID NO. 5 |
| ON6: 5'-(dA)₅₀ | SEQ ID NO. 6 |
| ON7: 5'-(dA)₈₀ | SEQ ID NO. 7 |
| ON8: 5'-T*T*C C*A T*G G*T G*G*C | SEQ ID NO. 8 |
| ON9: 5'-T*T*C A*C T*G T*G G*G*C | SEQ ID NO. 9 |
| ON10: 5'-T*G*G C*G C*C G*G G*C*C | SEQ ID NO. 10 |
| ON11: 5'-T*G*C C*G G*C C*G G*G*C | SEQ ID NO. 11 |

wobei * die Positionen angibt, an denen eine Phosphodiester Brücke durch eine Phosphorothioat-Internukleosidbrücke ersetzt worden ist.

Diese Sequenzen wurden zu den folgenden Konjugaten (KO)umgewandelt:

| | |
|---|---|
| KO_1: | F3-Li1-ON1 |
| KO_2: | F0-Li1-ON1 |
| KO_3: | F3-Li1-ON2 |
| KO_4: | F0-Li1-ON2 |
| KO_5: | F3-Li1-ON3 |
| KO_6: | F9-Li1-ON3 |
| KO_7: | F2-Li-1ON3 |
| KO_8: | F0-Li1-ON3 |
| KO_9: | F3-Li1-ON3-Rhodamin |
| KO_10: | F9-Li1-ON3-Rhodamin |
| KO_11: | F6-Li1-ON3-Rhodamin |
| KO_12: | F0-Li1-ON3-Rhodamin |
| KO_13: | F3-Li1-ON4 |
| KO_14: | F3-Li1-ON5 |
| KO_15: | F3-Li1-ON6 |
| KO_16: | F3-Li1-ON7 |
| KO_17: | F3-Li1-ON8 |
| KO_18: | F3-Li1-ON9 |
| KO_19: | F3-Li1-ON10 |
| KO_20: | F3-Li1-ON11 |
| KO_21: | F7-Li1-ON3 |

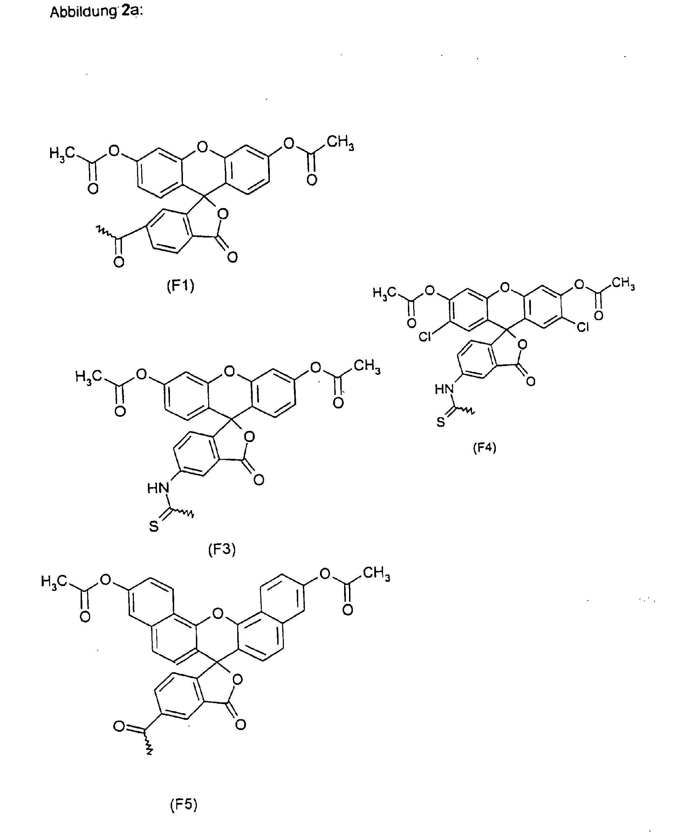

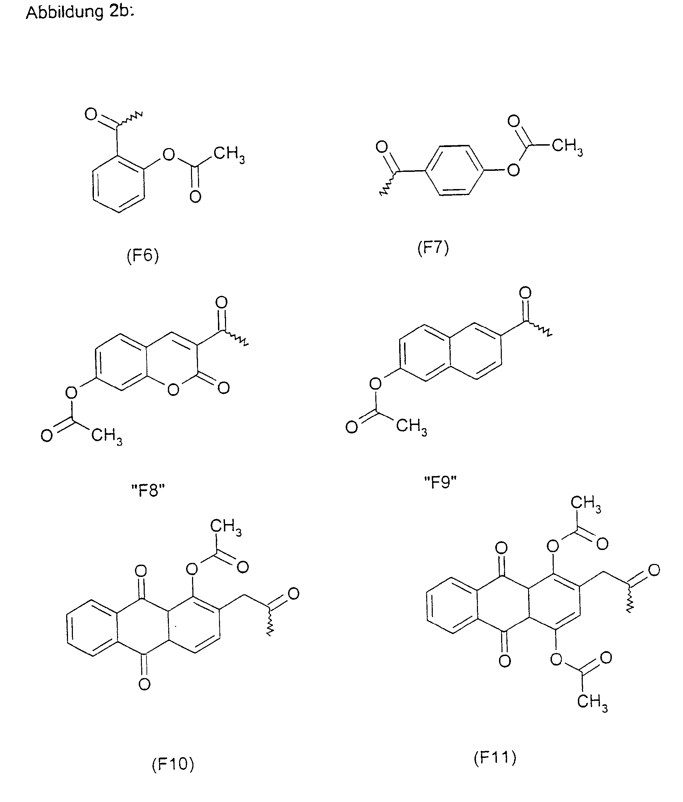

wobei
"F1 bis F11" Aryl-Reste der Formeln F1 bis F11 bedeuten (z.B. Abb. 2);
"Li1" ein 6-Aminohexylphosphat-Rest ist, der an das 5'-Ende des Oligonukleotids gebunden ist (z.B. Abbildung s. Anlage 4);
"ON1 bis ON11" die beschriebenen Oligonukleotide der Sequenzen SEQ ID NO.1 bis SEQ ID NO.11 bedeuten;
und "Rhodamin" eine neben Fluorescein nachweisbare Rhodamin-Markierung am 3'-Ende des Oligonukleotids ist.

Bei dem Verfahren zur Herstellung eines Konjugats, das ein zu transportierendes Molekül und mindestens einen Aryl-Rest der Formeln F1 und F3 bis F11 enthält, wird
a) ein zu transportierendes Molekül, das eine reaktive Funktion an der Position enthält, an die der Aryl-Rest gebunden werden soll, und
b) der Arylrest hergestellt und
c) das zu transportierende Molekül mit dem Aryl-Rest zum Konjugat umgesetzt.

Vorzugsweise ist die reaktive Funktion eine Aminogruppe, Mercaptogruppe, Chloracetylgruppe, Isocyanatgruppe, Isothiocyanatgruppe, Carbonsäuregruppe, N-Hydroxysuccinimidgruppe oder eine Carbonsäurechloridgruppe. Die Umsetzung des zu transportierenden Moleküls mit dem Aryl-Rest wird bei einem pH-Wert ≤ 7,5 durchgeführt; vorzugsweise bei einem pH-Wert ≤ 7,3, besonders bevorzugt bei einem pH-Wert von 7,0 oder einem niedrigeren pH-Wert, beispielsweise einem pH-Wert < 7, vorzugsweise pH-Wert ≤ 6,5 durchgeführt wird. Bei diesen Kupplungsreaktionen müssen alle anderen reaktiven Gruppen mit dem Fachmann bekannten Schutzgruppen vor der Reaktion geschützt werden.

In einer besonderen Ausführungsform der Verfahren ist das zu transportierende Molekül ein Polynukleotid, Oligonukleotid oder Mononukleotid.

Die Verfahren zu Herstellung beinhalten, daß in einem ersten Schritt das zu transportierende Molekül hergestellt wird. In diesem Zusammenhang betrifft die Erfindung auch Verfahren zur Herstellung von Oligonukleotiden. Die Oligonukleotide können mit Hilfe verschiedener bekannter, chemischer Verfahren, z.B. wie in Eckstein, F. (1991) "Oligonukleotides and Analogues, A Practical Approach", IRL Press, Oxford beschrieben, hergestellt werden. Die Oligonukleotide können auch durch Verfahren hergestellt werden, die gegebenenfalls einen oder mehrere enzymatische Schritte enthalten. Die Herstellung von Oligonukleotid-Konjugaten ist prinzipiell in der Literatur beschrieben (J. Goodchild, Bioconjugate Chem. 1 (1990) 165; S. Beaucage and R. lyer, Tetrahedron 49 (1993) 1925; S. Agrawal Mezhods in Molecular Biology Vol. 26 "Protocols for oligonucleotide Conjugates" (1994) Humana Press).

Bei der Synthese der Oligonukleotid-Konjugate gemäß Anspruch l ist jedoch darauf zu achten, daß diese sich im alkalischen Medium zersetzen können. Daher ist beispielsweise mit Hilfe der gängigen Methoden keine Synthese von FDA-markierten Oligonukleotide an einem Oilgonukleotid-Synthesizer möglich, da die Estergruppen der FDA-Gruppe bei der Ammoniakbehandlung, die zum Abspalten des Oligonukleotids vom Träger und der Abspaltung der Aminoschutzgruppen der heterozyklischen Basen notwendig ist, bereits hydrolysiert würden.
Daher wird das Oligonukleotid zunächst in entschützter Form als Vorstufe hergestellt und im letzten Schritt mit der Gruppe gemäß Anspruch l kondensiert (Abildung 5). Die Vorstufe des Oligonukleotids besitzt eine reaktive bzw. aktivierbare Funktion, die anschließend nach dem Fachmann bekannten Methoden mit einem Reagenz, das die erfindungsgemäße Gruppe gemäß Anspruch l enthält, derivatisiert wird. Als reaktive bzw. aktivierbare Funktionen kommen beispielweise Amino-, Mercapto-, Chloracetyl, Iso(thio)cyanat- und Carbonsäurefunktionen in Betracht.
Besonders leicht sind sogenannte Amino-Linker mit Hilfe kommerziell erhältlicher Reagenzien in Oligonukleotide einzuführen. Die Aminolinker-Oligonukleotide werden dann beispielsweise mit reaktiven Reagenzien, die eine Gruppe gemäß der Anspruch l enthalten, umgesetzt. Solche reaktiven Reagenzien sind beispielsweise die entsprechenden Isothiocyanate. Die Gruppe gemäß Anspruch l ist in diesem Falle über eine Thioharnstoff-Funktion verknüpft (Anlage 4). Andere reaktive Reagenzien sind beispielsweise die Carbonsäurechloride. Milde reaktive Reagenzien sind beispielweise die N-Hydroxysuccinimide der entsprechenden Carbonsäuren. Aktivierbare Reagenzien sind beispielsweise entsprechende Carbonsäuren, die sich mit Peptid-Kupplungsreagenzien wie HBTU, TBTU oder TOTU kuppeln lassen. In diesem Fall ist die Gruppe gemäß Anspruch l über eine Amidfunktion-Funktion verknüpft. Im Prinzip kann die Einführung der erfindungsgemäßen Gruppen der Anspruch l an beliebigen Positionen des Oligonukleotids erfolgen. Bevorzugt sind die in Abbildung 3 gezeigten Positionen.

Die Synthese der modifizierten Oligonukleotide erfolgte durch Aufbau der Oligonukleotid-Kette nach Standardmethoden, wie der Festphasensynthese nach der Phosphoramiditmethode, und Derivatisierung des 5'-Endes mit dem kommerziell erhältlichen 5'-Amino-Modifier C6 (z.B. Firma Eurogentec, Seraing, Belgien).

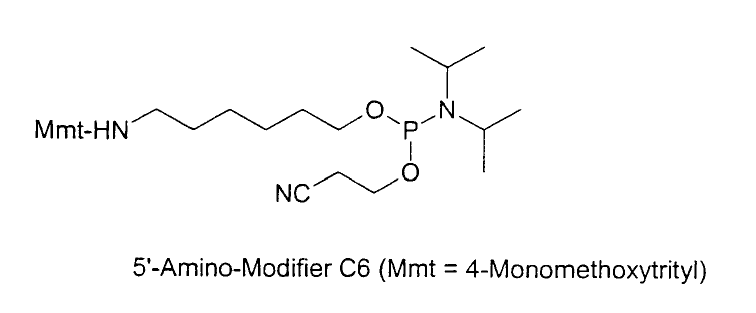

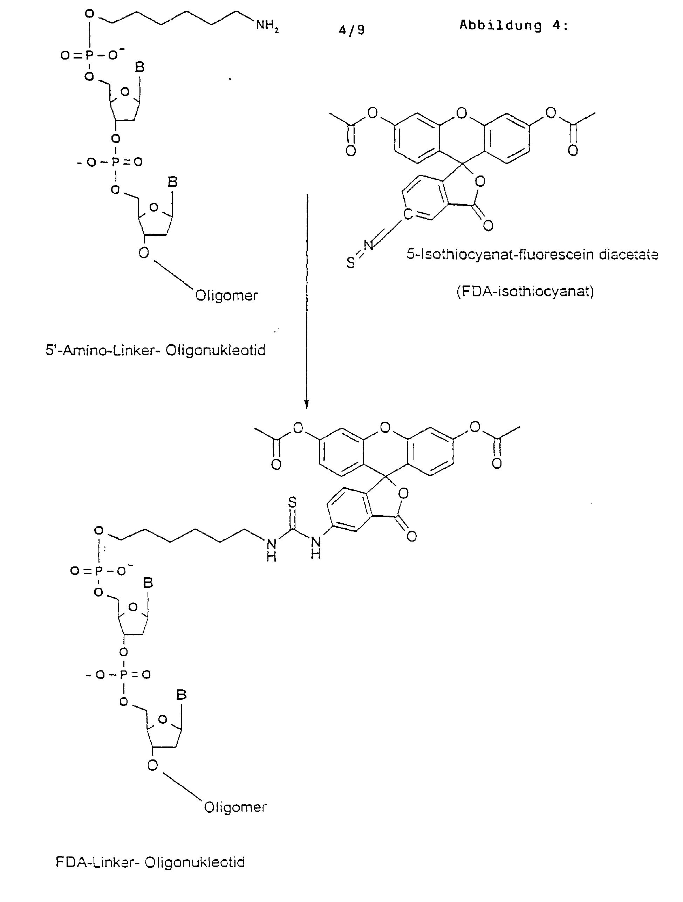

Nach Abspaltung des Oligonukleotid-Derivates vom Träger und Entschützung aller basenlabilen Schutzgruppen durch Behandlung mit Ammoniak, wird die Monomethoxytritylgruppe durch Behandlung mit 80% Essigsäure bei Umgebungstemperatur abgespalten. Man erhält ein 5'-Aminohexylphosphatmodifiziertes Oligonukleotid. Die Aminofunktion dieses Oligonukleotid-Derivats wird dann mit FDA-Isothiocyanat in 0.2 M Triethylammoniumbicarbonat Puffer (TBK-Puffer) pH 7 / DMF umgesetzt. Bereits nach etwa zwei bis drei Stunden war das Aminolinker-Oligonukleotid komplett zum gewünschten FDA-Derivat umgesetzt (Abbildung 4). Umsetzungen mit Fluorescein-isothiocyanat werden normalerweise bei pH 8 durchgeführt. Bei diesem pH-Wert wird allerdings das Diacetat der FDA-Gruppe hydrolysiert.
Natürlich können auch andere Aminolinker-Reagenzien eingesetzt werden, wie beispielsweise der 5'-Amino-Modifier C3, 5'-Amino-Modifier C12, 5'-Amino-Modifier 5 oder 5'-Thiol-Modifier C6 (alle von der Firma Eurogentec).

Durch den Einsatz von 3'-Amino-Modifier-Festphasen, wie beispielsweise 3'-Amino-Modifier C3 CPG (Firma Eurogenmtec) können Oligonukleotid-Derivate mit einer 3'-Aminoalkly-Gruppe hergestellt werden, die anschließend mit FDA-isothiocyanat zur Reaktion gebracht werden. man erhält ein Oligonucletid-Derivat, das die 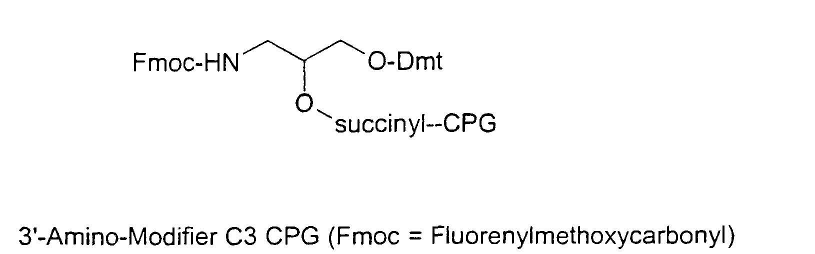 erfindungsgemäße Gruppe der Formel I am 3'-Ende gebunden hält.

Zur Einführung des Konjugates an der heterocyclischen Base des Nucleosids kann man ein entsprechendes Amino-Modifier C6 dT (Firma Eurogentec), das sich von Thymidin ableitet, in der Synthese anstelle eines normalen Phosphoramidit-Bausteins einsetzen. Am Ende der Oligonukleotid-Syhtese wird die Trifluoracetyl-Schutzgruppe durch Ammoniakbehandlung abgespalten und die freie Aminofunktion in Lösung mit FDA-isothioacyanat zur Reaktion gebracht.

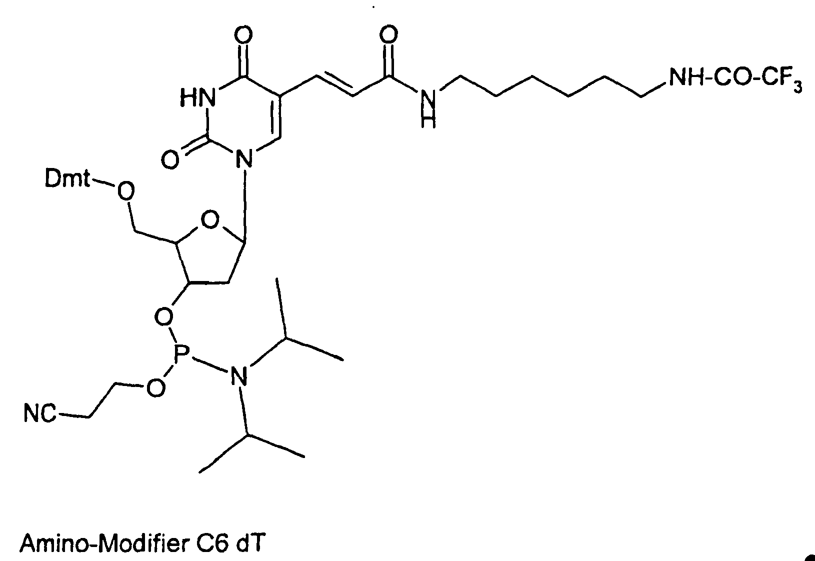

In ähnlicher Weise können die erfindungsgemäßen Gruppen der Anspruch l in beliebige Positionen der Oligonukleotide eingeführt werden. Es ist leicht ersichtlich, daß auch eine mehrfache Einführung von gleichen oder unterschiedlichen Gruppen möglich ist.

Bei der erfindungsgemäßen Verwendung ist die biologische Membran vorzugweise Bestandteil einer Zelle, eines Vesikels oder einer Organelle.

Gegenstand der vorliegenden Erfindung sind auch Verfahren zum Transport eines Moleküls über eine Membran in vitro, wobei ein Arylrest, wie oben beschrieben der an ein zu transportierendes Molekül ausgewählt aus einem Polynukleotid, Oligonukleotid oder Mononukleotid gebunden ist mit der Membran inkubiert wird.

Insbesondere, Verfahren zum Transport eines Moleküls in eine Zelle in vitro, wobei ein Arylrest, wie oben beschrieben der an ein zu transportierendes Molekül ausgewählt aus einem Polynukleotid, Oligonukleotid oder Mononukleotid gebunden ist mit der Zelle inkubiert wird, woraufhin das Konjugat in die Zelle transportiert wird, ohne daß der Aryl-Rest abgespalten wird.

Dies betrifft insbesondere Verfahren, in denen die Zelle eine eukaryotische oder eine prokaryotische Zelle, beispielsweise eine Bakterienzelle, Hefezelle oder eine Säugetierzelle, vorzugsweise eine humane Zelle ist. In besonderen Ausführungsformen ist die Zelle eine pathologisch veränderte Zelle, z.B. eine Tumorzelle.

Die verbesserte zelluläre Aufnahme der Konjugate wurde nicht nur bei Zellen von Säugetieren beobachtet, sondern auch für andere Eukaryoten und sogar für Prokaryoten nachgewiesen.

Die erfindungsgemäßen Konjugate wurden mikroskopisch auf die Aufnahme in lebende Zellen untersucht. Zunächst wurden die FDA-markierten Oligonukleotide auf KO_1 und KO_3 Zellgängigkeit untersucht. Als dem Stand der Technik bekannte Verbindungen wurden die entsprechenden Fluorescein-markierten Oligonukleotide KO_2 und KO_4 eingesetzt. Alle untersuchten vitalen tierischen Zellkulturen nahmen KO_1 und KO_3 (FDA-Konjugate) innerhalb von 5 bis 10 Minuten auf, während KO_2 und KO_4 (Fluorescein-Konjugate) nach diesen Zeit nicht in vitalen Zellen nachweisbar war (Tabelle 1)

Obwohl die Aufnahme in Bakterien und Hefe viel langsamer erfolgt als in in Säugerzellen, hat nach zwei Stunden doch eine Aufnahme der erfindungsgemäßen Oligonukleotide in einen Teil der Zellen stattgefunden, während die normalen Fluorescein-markierten Oligonukleotide unter diesen Bedingungen nicht aufgenommen werden. Es ist erstaunlich, daß im Prinzip alle bislang untesuchten Organismen die erfindungsgemäßen Oligonukleotide besser als bekannte Oligonukleotid-Derivate aufgenommen haben. Die Organismen umfassen unter anderem tierische Zellen, Flagellaten, Hefen, Pilze und Bakterien (Tabelle 3).

Es zeigte sich ferner, daß Krebszellen die Oligonukleotide besonders gut aufnehmen. Daher ist die Anwendung der erfindungsgemäßen Oligonukleotide besonders für die Tumortherapie geeignet. Das FDA-markierte Antisense Oligonukleotid KO_1, das gegen eg5 gerichtet ist, hemmte die Proliferation von A549 Zellen bereits durch Zugabe ins Medium, während das entsprechende unmodifizierte Antisense Oligonucleotid ON 1 und das Fluorescein-markierte Oligonukleotid KO_2 nur nach Formulierung mit Penetrationsenhancern wie CellFectin die Proliferation der Krebszellen hemmten.

Die Erfindung betrifft die Verwendung von Konjugaten, in denen das zu transportierende Molekül ein Oligonukleotid ist, zur Hybridisierung mit einzelsträngigen und/oder doppelsträngigen Nukleinsäuren, beispielsweise DNA (z.B. Gene, cDNA) und/oder RNA (z.B. pre-mRNA, mRNA). Diese Konjugate können auch sequenzspezifisch an intrazelluläre Proteine wie Enzyme, beispielsweise Polymerasen oder Telomerasen, oder an Transkriptionsfaktoren binden. Die Erfindung betrifft weiterhin die Verwendung solcher Konjugate zur Modulation sowie zur ganzen oder teilweisen Inhibition der Expression von bestimmten Target-Genen, beispielsweise zur ganzen oder teilweisen Inhibition der Transkription und/oder der Translation. Die Erfindung betrifft auch die Verwendung solcher Konjugate als Antisense Oligonukleotide, Ribozyme, Sense Oligonukleotide, Tripelhelix-bildende Oligonukleotide, Chimeraplasten und/oder Decoy-Oligonukleotide. Darüber hinaus können diese Konjugate als Hilfsmittel in der Molekularbiologie verwendet werden.

Die Erfindung betrifft weiterhin die Verwendung der Oligonukleotide als Arzneimittel und/oder Diagnostikum bzw. die Verwendung der Oligonukleotide zur Herstellung von Arzneimitteln und/oder Diagnostika. Insbesondere können die Oligonukleotide in Arzneimitteln, die zur Prävention und/oder Behandlung von Krankheiten, die mit der Expression bzw. einer Überexpression bestimmter Gene einhergehen, eingesetzt werden. Weiterhin können die Oligonukleotide für die Diagnose bzw. Früherkennung solcher Krankheiten eingesetzt werden. Da die Zellgängigkeit der erfindungsgemäßen Oligonukleotide sehr gut ist, können diese für die in vivo Diagnostik, beispielsweise zur in situ Hybridisierung an ganzen Organen oder am intakten Organsimus, eingesetzt werden.

Die Erfindung betrifft auch die Verwendung der Oligonucleotide zur Detektion, Separation und Amplifikation von Nucleinsäuren und deren Analoga. Die Konjugate eignen sich besonders zum Nachweis von Nucleinsäuren in Zellen, insbesondere in lebenden Zellen. Diese Zellen können humanen oder tierischen Ursprungs sein. Besonders eignen sich die Konjugate auch für die in Tabelle 3 aufgeführten Organismen, insbesondere zum Nachweis von pathogenen Organismen. Die erfindungsgemäßen Oligonucleotide können in bekannte technische Varianten der Amplifikation von Nucleinsäuren eingesetzt werden, insbesondere in die LMPCR (ligation-mediated Polymerase Chain Reaction), in den "Invader Assay " (Warenzeichen, Third Wave technologies, Inc., Wisconsin), im TaqMan System (Warenzeichen) und im Multiplex Genotyping. Vorteilhaft ist auch die Verwendung der Oligonukleotide zur Amplifikation von Nukleinsäuren mit Hilfe des Light-Cyclers, das eine Echtzeit- Bestimmung der Amplifikation erlaubt. Die Detektion nach dem Prinzip der molekularen "Beacons", bei denen der Fluoreszenzfarbstoff im ungebundenen Zustand nicht fluoresziert, weil er durch eine zweite Gruppe im Oligomer gequencht wird, ist eine weitere Einsatzmöglichkeit der erfindungsgemäßen Oligonukleotide. Beispielsweise kann ein FDA-Derivat (z.B. am 5'-Ende des Oligonukleotids) mit einem Dabcyl-Rest (z.B. am 3'-Ende konjugiert) kombiniert werden, der auch nach Umwandlung des FDA-Derivats in das Fluorescein-Derivat das Fluoreszenzsignal im ungebundenen Zustand quencht. Diese FDA-modifzierten Beacons würden erst nach Aufnahme in die Zelle und Hybridisierung an die Ziel-mRNA ein Fluoreszenzsignal aussenden.
Die Erfindung betrifft auch die Verwendung der Oligonukleotide bzw. von Arzneimitteln, die diese Oligonukleotide enthalten, zur Behandlung von Krankheiten, bei denen definierte Gene durch Überexpression ursächlich bzw. beteiligt ist.
Die Arzneimittel der vorliegenden Erfindung können beispielsweise zur Behandlung von Erkrankungen, die durch Viren hervorgerufen werden, beispielsweise durch CMV, HIV, HSV-1, HSV-2, Influenza, VSV, Hepatitis B oder Papilloma Viren, verwendet werden. Die Arzneimittel der vorliegenden Erfindung eignen sich beispielsweise auch zur Behandlung von Krebs. Die Arzneimittel der vorliegenden Erfindung eignen sich beispielsweise ferner zur Behandlung von Erkrankungen, die durch Integrine oder Zell-Zell-Adhäsionsrezeptoren beeinflußt werden, beispielsweise durch VLA-4, VLA-2, ICAM, VCAM oder ELAM. Die Arzneimittel der vorliegenden Erfindung eignen sich beispielsweise auch zur Verhinderung der Restenose, zur Behandlung von Vitiligo und anderen Depigmentierungskrankheiten bzw. Depigmentierungsstörungen (z.B. der Haut, Haare, Augen) beispielsweise Albinismus und Psoriasis, von Asthma.

Die Arzneimittel betreffen z.B. pharmazeutische Präparate, die man a) oral, z.B. in Form von Tabletten, Dragees, Hart- oder Weichgelatinekapseln, Lösungen, Emulsionen oder Suspensionen, b) rektal, z.B. in Form von Suppositorien oder c) parenteral, z.B. in Form von Injektionslösungen verabreichen kann. Für die Herstellung der Arzneimittel können die Konjugate z.B. in therapeutisch inerten organischen und/oder anorganischen Trägern verarbeitet werden; beispielsweise können als Träger für Tabletten, Dragees und Hartgelatinekapseln Laktose, Maisstärke oder Derivate davon, Talg und Stearinsäure oder Salze davon verwendet werden. Als Träger für Lösungen sind Wasser, Polyole, Saccharose, Invertzucker und Glucose, für Injektionslösungen sind Wasser, Alkohole, Polyole, Glycerol und pflanzliche Öle, für Suppositorien sind pflanzliche und gehärtete Öle, Wachse, Fette und halbflüssige Polyole geeignet. Die Arzneimittel können zudem Konservierungsmittel, Lösemittel, Stabilisierungsmittel, Netzmittel, Emulgatoren, Süßstoffe, Farbstoffe, Geschmacksmittel, Salze zur Veränderung des osmotischen Drucks, Puffer, Überzugsmittel, Antioxidantien, sowie ggf. andere therapeutische Wirkstoffe enthalten. Vorzugsweise werden die Arzeimittel topisch appliziert oder lokale appliziert wie beispielsweise mit Hilfe eines Katheters oder inhaliert, oder per Injektionen bzw. Infusionen verabreicht. Zur Injektion wird das Konjugat in einer flüssigen Lösung, vorzugsweise in einem physiologisch annehmbaren Puffer, wie z.B. Hank's Lösung oder Ringer's Lösung, formuliert. Das Konjugat kann aber auch in fester Form formuliert werden und vor dem Gebrauch gelöst oder suspendiert werden. Die für die systematische Verabreichung bevorzugten Dosierungen betragen ca. 0,01 mg/kg bis ca. 50 mg/kg Körpergewicht und Tag.
Die Konjugate und/oder deren physiologisch verträglichen Salze können am Tier, bevorzugt am Säugetier, und insbesondere am Menschen als Arzneimittel für sich allein, in Mischungen untereinander oder in Form von pharmazeutischen Zubereitungen verabreicht werden, die eine topische, perkutane, parenterale oder enterale Anwendung gestatten und die als aktiven Bestandteil eine wirksame Dosis mindestens eines Konjugats, neben üblichen pharmazeutisch einwandfreien Träger- und Zusatzstoffen enthalten. Die Zubereitungen enthalten normalerweise etwa 0,1 bis 90 Gew.-% der therapeutisch wirksamen Verbindung. Zur Behandlung von Hautkrankheiten, wie beispielsweise Psoriasis oder Vitiligo wird eine topische Anwendung, z.B. in Form von Salben, Lotionen oder Tinkturen, Emulsionen, Suspensionen bevorzugt. Die Herstellung der Arzneimittel erfolgt in an sich bekannter Weise, (z. B. Remingtons Pharmaceutical Sciences, Mack Publ. Co., Easton, PA.), wobei pharmazeutisch inerte anorganische und/oder organische Trägerstoffe verwendet werden. Für die Herstellung von Pillen, Tabletten, Dragees und Hartgelatinekapseln kann man z.B. Lactose, Maisstärke und/oder Derivate derselben, Talk, Stearinsäure und/oder deren Salze etc. verwenden. Trägerstoffe für Weichgelatinekapseln und/oder Suppositorien sind z.B. Fette, Wachse, halbfeste und flüssige Polyole, natürliche und/oder gehärtete Öle etc. Als Trägerstoffe für die Herstellung von Lösungen und/oder Sirupen eignen sich z.B. Wasser, Saccharose, Invertzucker, Glukose, Polyole etc. Als Trägerstoffe für die Herstellung von Injektionslösungen eignen sich Wasser, Alkohole, Glycerin, Polyole, pflanzliche Öle etc. Als Trägerstoffe für Mikrokapseln, Implantate und/oder Rods eignen sich Mischpolymerisate aus Glykolsäure und Milchsäure. Darüber hinaus sind Liposomenformulierungen, die dem Fachmann bekannt sind, geeignet (N. Weiner, Drug Develop Ind Pharm 15 (1989) 1523; "Liposome Dermatics, Springer Verlag 1992), beispielsweise HVJ-Liposomen (Hayashi, Gene Therapy 3 (1996) 878).

Ein Arzneimittel kann neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungsmittel, Puffersubstanzen, ferner Lösungsmittel und/oder Lösungsvermittler und/oder Mittel zur Erzielung eines Depoteffekts, sowie Salze zur Veränderung des osmotischen Drucks, Überzugsmittel und/oder Antioxidantien enthalten. Sie können auch zwei oder mehrere verschiedene Oligonukleotide und/oder deren physiologisch verträgliche Salze enthalten sowie ferner neben mindestens einem Oligonukleotid einen oder mehrere andere therapeutisch wirksame Stoffe. Die Dosis kann innerhalb weiter Grenzen variieren und ist in jedem einzelnen Fall den individuellen Gegebenheiten anzupassen.

### Abbildungen

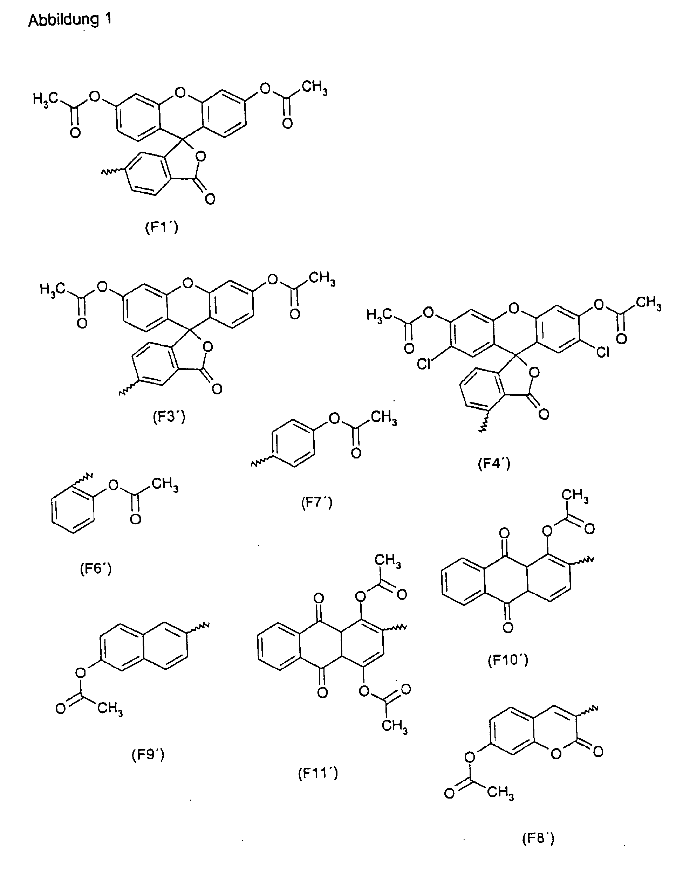

Abbildung 1: Die Abbildung zeigt Beispiele für Aryl-Reste.

Abbildung 2 a und b : Die Abbildung zeigt Beispiele für Aryl-Reste.

Abbildung 3: Abbildung 3 zeigt verschiedene Beispiele (A, B, C, D, E, F, G) für eine Konjugation zwischen einem zu transportierenden Molekül (hier ein Oligonukleotid) und Aryl-Resten des Anspruchs I. "R" steht für einen Rest des Anspruchs I; "B" steht für eine in der heterozyklische Base.

Abbildung 4: Abbildung 4 zeigt eine Möglichkeit, ein erfindungsgemäßes Konjugat (hier bestehend aus FDA-isothiocyanat und Oligonukleotid) herzustellen.

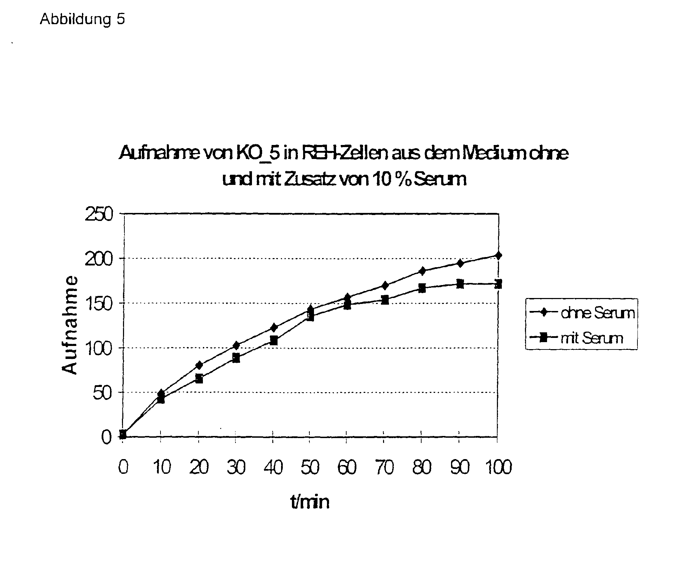

Abbildung 5: Abbildung 5 zeigt den zeitlichen Verlauf der Aufnahme des Konjugats KO_5 in REH-Zellen aus dem Medium, wobei einmal Medium ohne Serum (◆) und einmal Medium mit Serum (■) verwendet wurde. Die Zellaufnahme wurde mit Hilfe von FACS bestimmt.

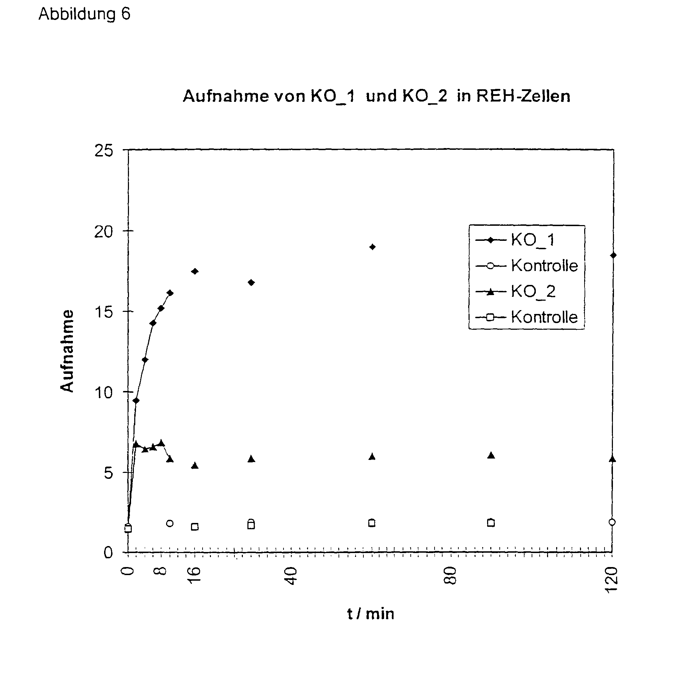

Abbildung 6: Abbildung Bestimmung der Zellaufnahme von KO_1 (FDA-Konjugat; ◆) und KO_2 (FITC-Oligomer; ▲) durch FACS-Messung. Die Anfangskonzentration an extrazellulärem Oligonukleotid-Konjugat betrug 1 µM; ○ und □ sind Kontrollen.

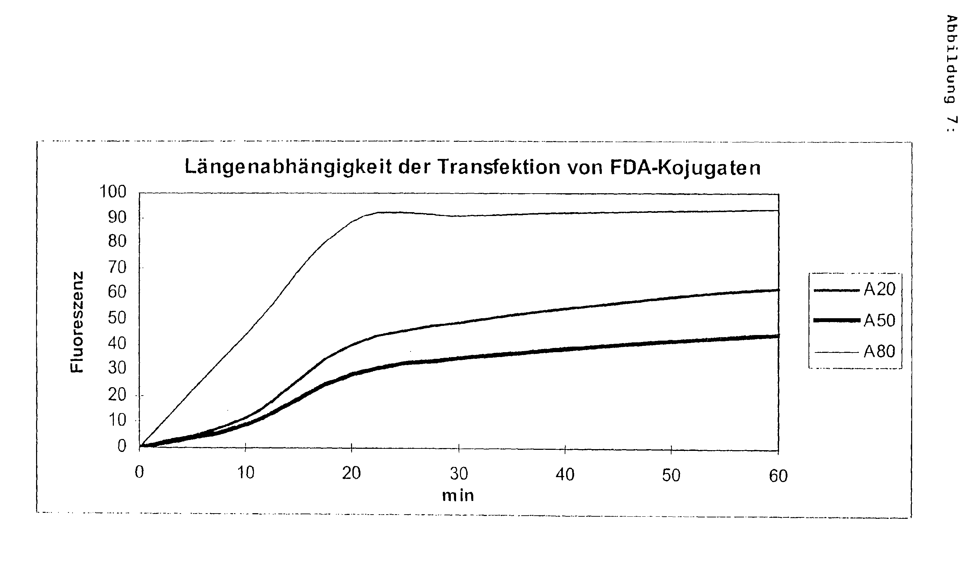

Abbildung 7: Längenabhängigkeit der Transfektion von FDA-konjugierten polyA-Nukleotiden in REH-Zellen

Abbildung 8: Vergleich der Zellaufnahme von Fluorescein-Diacetat- und Fluorescein-Dipivalat Oligonucleotid-Konjugaten
K39: Carboxyfluorescein-Diacetat (F3 gleich FDA) aus Beispiel 15
K41: Carboxyfluorescein-Dipivalat (F2) aus Beispiel 10

Abbildung 9: Fluoreszenzmikroskopische Untersuchung der Zellaufnahme des zweifach markierten Cy3-Oligonucleotid-F3-Konjugats [1 µM] bei der Inkubation mit REH-Zellen. Aufnahme nach 4 Stunden. links: Fluoreszenzmarkierung; Mitte: Cyanin Farbstoff; Rechts: Phasenkontrastaufnahme; Oben: Oligonukleotid K33; Unten: Oligonukleotid K34.

### Beispiele

### Beispiel 1: Oligonukleotidsynthese

Oligonukleotide wurden auf einem automatischen DNA Synthesizer (Applied Biosystems Model 380B oder 394) unter Anwendung der Standard Phosphoramidit-Chemie und Oxidation mit Jod synthetisiert (F. Eckstein, Ed "Oligonukleotides and Analogues, A Practical Approach", IRL Press, Oxford, 1991). Zur Einführung von Phosphorthioat-Brücken in gemischten Phosphorothioaten und Phosphodiester Oligonukleotiden wurde anstelle von Jod mit TETD (Tetraethylthiuramdisulfid) oxidiert oder Beaucage's Reagenz oxidiert. Nach Abspaltung vom festen Träger (CPG oder Tentagel) und Entfernung der Schutzgruppen mit konz. NH₃ bei 55°C während 18h, wurden die Oligonukleotide zunächst durch Butanol-Fällung (Sawadogo, Van Dyke, Nucl. Acids Res. 19 (1991) 674 ) gereinigt. Die Oligonukleotide wurden durch präparative Gelelektrophorese oder FPLC gereinigt. Anschließend wurde das Natriumsalz erhalten durch Ausfällung aus einer 0.5 M NaCl Lösung mit 2.5 Volumenteilen Ethanol.

Die Analyse der Oligonukleotide erfolgte durch
a) Analytische Gelelektrophorese in 20% Acrylamid, 8M Harnstoff, 454M Tris-borat Puffer, pH 7.0 und/oder
b) HPLC-Analyse: Waters GenPak FAX, Gradient CH₃CN (400ml), H₂O (1.6l), NaH₂PO₄ (3.1 g), NaCl (11.7g), pH6.8 (0.1M an NaCl) nach CH₃CN (400ml), H₂O (1.61), NaH₂PO₄ (3.1g), NaCl (175.3g), pH6.8 (1.5M an NaCl) und/oder
c) Kapillargelelektrophorese Beckmann Kapillare eCAP^{™}, U100P Gel Column, 65 cm length, 100 mm I.D., window 15 cm from one end, Puffer 140 µM Tris, 360mM Borsäure, 7M Harnstoff und/oder
d) Elektrospray Massenspektroskopie

Die Analyse des Oligonukleotids ergab, daß dieses jeweils in einer Reinheit von größer 90%, meistens aber größer 95% vorlag.

### Beispiel 2: Einführung eines 5'-Amino-Linkers in ein Oligonukleotid

Das Oligonukleotid wurde wie in Beispiel 1 beschrieben aufgebaut. Nach Kupplung des letzten Nukleotids wurde die Dimethoxytritylgruppe am 5'-Ende abgespalten. Die freie Hydroxylgruppe wurde mit dem kommerziell erhältlichen 5'-Amino-Modifier C6 (Firma Eurogentec, Seraing, Belgien) unter Tetrazol-Katalyse umgesetzt, mit Jodwasser oxidiert. Dann wurde das Oligonukleotid durch Behandlung mit conc. Ammoniak bei 50°C über Nacht vom Träger gespalten und alle basenlabilen Schutzgruppen an den Internucleosid-Gruppen und den Aminiofunktionen der heterocyclischen Basen abgespalten. Im letzten Schritt wurde die Monomethoxytrityl-Schutzgruppe durch 3-stündige Behandlung mit 80%-iger Essigsäure bei Umgebungstemperatur abgespalten. Das resultierende Oligonukleotid wurde wie in Beispiel 1 beschrieben analysiert.

### Beispiel 3: Konjugation des Amino-Linker Oligonukleotids mit FDA-Isothiocyanat

### (Anschanungsbeispiel)

10 OD (260) des 5'-Amino-Linker Oligonukleotids aus Beispiel 2 wurden in 16 µl 0.2 M Triethylammoniumbicarbonat (TBK) Puffer gelöst und mit 125 µl Dimethylformamid (DMF) versetzt. Zu dieser Mischung gab man 1.5 mg FDA-Isothiocyanat zu und ließ diese dann 3 Stunden uner Lichtausschluß schütteln. Der Erfolg der Umsetzung wurde durch HPLC überprüft. danach wurden 2 µl conc. Essigsäure zugegeben und im Vakuum konzentriert. Anschließend wurde das Produkt durch eine Butanolfällung gereinigt. Mit Hilfe der ESI-Massenspektroskopie wurde das richtige Massengewicht festgestellt. Die Proben wurden stets bi pH kleiner 7 gehalten, damit keine Hydrolyse des aromat Esters stattfand.

### Beispiel 4: Synthese von KO_1 (5'-F3-G*CGAC*GC*CAT*GAC*G*G-3' ;F3 = FDA)

Das Oligonucleotid wurde ausgehend von einem CPG-Träger, der 1 µmol Desoxyguansoin über das 3'-Ende gebunden hielt, wie in Beispeil 1 beschrieben aufgebaut. An den mit * gekennzeichneten Positionen wurde die Oxidation mit Beaucage Reagenz durchgeführt, um eine Phosphorothioat-Brücke einzuführen. Dann wurde der 5'-Amino-Modifier C6 wie in Beispiel 2 beschrieben aufgekuppelt. Nach der Entschützung mit conc. Ammoniak und 80% Essigsäure erhielt man 96 OD (260) des 5'-Aminolinker-G*CGAC*GC*CAT*GAC*G*G-3'.
10 OD (260) des 5'-Amino-Linker-Oligonucleotids wurden dann wie in Beispiel 3 beschrieben mit FDA-Isothiocanat umgesetzt. Nach der Butanolfällung erhielt man 8.4 OD (260) des gewünschten FDA-markierten Oligonucleotids. ESI-MS für di-Na-Salz: 5395.93 (berechnet für di-Na: 5395.09)

### Beispiel 5: Synthese von KO_13 (5'-F3-TATTCCGTCAT-3')

Das Oligonucleotid wurde ausgehend von einem CPG-Träger, der 1 µmol Thymidin über das 3'-Ende gebunden hielt, wie in Beispeil 1 beschrieben aufgebaut. Alle Oxidationen wurden mit Jodwasser durchgeführt. Dann wurde der 5'-Amino-Modifier C6 wie in Beispiel 2 beschrieben aufgekuppelt. Nach der Entschützung mit conc. Ammoniak und 80% Essigsäure erhielt man 72 OD (260) des 5'-Aminolinker-TATTCCGTCAT-3'. nach der Reinigung über ein präparatives Polyacrylamid-gel erhielt man 43 OD (260).
10 OD (260) des 5'-Amino-Linker-Oligonucleotids wurden dann wie in Beispiel 3 beschrieben mit FDA-Isothiocanat umgesetzt. Nach der Butanolfällung erhielt man 9.1 OD (260) des gewünschten FDA-markierten Oligonucleotids. ESI-MS: 3934.1 (berechnet MW 3933.8).

### Beispiel 6: Synthese von KO_21 (5'-F7-A*T*G A C*G G A A*T*T*C)

Das Oligonucleotid wurde ausgehend von einem CPG-Träger, der 1 µmol N6-Benzoylcytidin über das 3'-Ende gebunden hielt, wie in Beispeil 1 beschrieben aufgebaut. Alle Oxidationen wurden mit Jodwasser durchgeführt. Dann wurde der 5'-Amino-Modifier C6 wie in Beispiel 2 beschrieben aufgekuppelt. Nach der Entschützung mit conc. Ammoniak und 80% Essigsäure erhielt man 145 OD (260) des 5'-Aminolinker-A*T*G A C*G G A A*T*T*C-3'.

10 OD (260) des 5'-Amino-Linker-Oligonucleotids wurden in 16 µl 0.2 M TBK-Puffer, 95 µl DMF gelöst und mit 30 µl des zuvor hergestellten Aktivesters der p-Acetoxybenzoesäure umgesetzt. Die Herstellung des Aktivesters erfolgte durch Mischen von 50 µl 0.2 M p-Acetoxybenzoesäure mit 50 µl 0.3 M TBTU, jeweils in DMF, und einstündige Reaktion bei Umgebungstemperatur. Nach 4 Stunden Reaktion des Aminolinker-Oligonucleotids mit dem Aktivester gibt man 2µl halbkonzentrierte Essigsäure zu und konzentriert im Vakkum. Überschüssiges Reagenz wurde durch eine Butanol-Fällung entfernt. Man erhielt 10.7 OD (260) des gewünschten Oligonucleotid-Konjugats. ESI-MS: 4109.2 (berechnet MW 4108.2).

### Beispiel 7: Untersuchung der zellulären Aufnahme der Oligonucleotid-Konjugate:

Zur Untersuchung der Zellaufnahme wurden 1ml Zellsuspension in einer Bachofer-Kammer in Kulturmedium (oder nach Spülen in PBS bei Medien mit Eigenfluoreszenz) unter mikroskopischer Kontrolle mit 1ml einer 1 µmolaren Lösung des Oligonucleotid-Konjugates versetzt, wobei die Durchmischung mit der Pipette und durch Schütteln der Kammer erfolgte. Die Mikroskopie wurde mit Hilfe des Zeiss Axiovert 135 TV Geräts (100 x Plan-Neofluar) im Phasenkontrast-Modus durchgeführt. Als Fluoreszenz-Filter dienten 09 (450-490/ FT 510/ LP 520) / HBO 59W Filter. Als Referenz wurde eine 2.4 µM Lösung von FDA (Aldrich Chem. Co, FW.416.39) in Acteon/PBS-Puffer (1: 1000; v:v) eingesetzt. Im Falle der FDA-Konjugate kann nach Aufnahme die Eigenfluoreszenz des durch Esterspaltung entstandenen Fluoescein-Liganden verfolgt werden. Im Falle nichtfluoreszierender Liganden wie Acetoxynaphthalin-Carbonsäure wurde zusätzlich ein geeigneter Fluoreszenz-Label (FITC, Rhodamin, Cyaninfarbstoff Dy 3 oder Dy5) am Oligonucleotid angebracht. Eine Doppelmarkierung wie in KO_9 diente dazu zu zeigen, daß FDA nicht vom Oligonucleotid abgespalten wurde. Die einzelnen Proben wurden 2 bis 120 Minuten nach Zugabe des Oligonucleotid-Konjugats bewertet. Bei Reh-Zellen war die Fluoreszenz nach 5 bis 10 min deutlich zu erkennen, bei K562 und adhaerenten Zellen sowie Insektenzellen erfolgte eine gewisse Zunahme noch bis zu 60 min nach Zugabe. Bei freilebenden Protozoen dauerte die Aufnahme bis zu 1 h. Bei Hefen erfolgte die Aufnahme erst nach längerer Zeit und nicht homogen in alle Zellen. Pilzsporen nahmen FDA-Oligonucleotid-Konjugate besser auf als Hyphenzellen. Die Ergebnisse sind in den Tabellen 1 bis 3 zusammengefaßt.

### Beispiel 8: Untersuchung der antiproliferativen Wirkung der Oligonucleotid-Konjugate.

Die REH-Zellen (humane prae-B Zell-Leukämie, DSM ACC 22) oder A549 Tumorzellen wurden inOptiMEM mit 10 % Fötalem Kälberserum (FKS;GIBCO-BRL) bei 37°C unter 5% CO₂ kultiviert. Am Tage vor dem Versuchsansatz wurden die Zellen so umgesetzt, daß nach 24 h eine Zellkonzentration von etwa 1 x 10⁶ / ml erreicht werden konnte. Die Oligonucleotide bzw. deren Konjugate wurden in destilliertem Wasser als 1 mM Stammlösungen gelöst und im Kühlschrank bei -20°C aufbewahrt. Die Zelleinsaat: (1 x 10⁶ Zellen / ml in OptiMEM mit 10 % FKS) erfolgte in 24-Lochplatten. Zur Untersuchung wurden die Oligonucleotid-Derrivate auf 2µM (in OptiMEM ohne FKS) verdünnt. Pro Loch wurden 100 µl Oligonucleotidlösung und 100 µl Zellsuspension gemischt (Gesamtvolumen 200µl / Loch; Oligokonzentration 1µM, Serumkonzentration 5 % FKS, Zellkonzentration 0,5 x 10⁶ Zellen / ml). Nach 4 h Inkubation bei 37°C und 5% CO₂ wurden pro Loch 800 µl OptiMEM mit 11 % FKS zugegeben (Zellkonzentration jetzt 1x10⁵ Zellen / ml, Serumkonzentration jetzt 10% FKS) und weiter inkubiert. Nach 96 h bei 37 °C und 5% CO₂ erfolgte die Messung der Zellkonzentration am Casy 1 (Fa. Schärfe). Hierzu wurden die Zellen in jedem Loch durch 10 -maliges Ansaugen und Ausblasen mit 1000er Pipette gemischt und sofort 1:100 (bei stärkerem Zellwachstum 1:200) mit Casyton verdünnt. Es erfolgte eine Mittelwertbestimmung der Zelldichte aus jeweils 3 identisch angesetzten Proben eines Versuchsansatzes.

### Beispiel 9: Synthese von 5'-F4'(CO-NH)-A*T*G A C*G G A A*T*T*C

### (Anschanungsbeispiel)

Das Oligonucleotid wurde ausgehend von einem CPG-Träger, der 1 µmol N4-Benzoylcytidin über das 3'-Ende gebunden hielt, wie in Beispiel 1 beschrieben aufgebaut. Die Oxidationen wurden mit Jodwasser bzw. Beaucage-Reagenz zur Einführung eines Phosphorothioatrestes (wenn * in der Sequenz) durchgeführt. Dann wurde der 5'-Amino-Modifier C6 wie in Beispiel 2 beschrieben aufgekuppelt. Nach der Entschützung mit conc. Ammoniak und 80% Essigsäure erhielt man 145 OD (260) des 5'-Aminolinker-A*T*G A C*G G A A*T*T*C-3'.
10 OD (260) des 5'-Amino-Linker-Oligonucleotids wurden in 16 µl 0.2 M TBK-Puffer, 95 µl DMF gelöst und mit 12.5 µl des Dichlorfluoresceindiacetat-Hydroxysuccinimids (MW: 626.36) umgesetzt. Nach 3 Stunden Reaktion des Aminolinker-Oligonucleotids mit dem Hydroxysuccinimid gibt man 2µl halbkonzentrierte Essigsäure zu und konzentriert im Vakuum. Nach Entsalzung über eine NAP-Säule™ (Pharmacia) wurde eine Butanol-Fällung durchgeführt. Man erhielt 2.8 OD (260) des gewünschten Oligonucleotid-Dichlorfluoresceindiacetat-Konjugats. ESI-MS: 4403.3 (berechnet MW 4403.2).

### Beispiel 10: Synthese von 5'-F2'-(CO-NH)-A*T*G A C*G G A A*T*T*C

### (Anschanungsbeispiel)

Das 5'-Aminolinker-A*T*G A C*G G A A*T*T*C-3' Oligomer wurde wie in Beispiel 9 beschrieben hergestellt. 10 OD (260) des 5'-Amino-Linker-Oligonucleotids wurden in 16 µl 0.2 M TBK-Puffer, 95 µl DMF gelöst und mit 12.5 µl des Carboxyfluoresceindipivalat-Hydroxysuccinimids (MW: 641.64) umgesetzt. Nach 2 Stunden Reaktion des Aminolinker-Oligonucleotids mit dem Hydroxysuccinimid gibt man nochmals 12.5 µl des Hydroxysuccinimids zu und lässt weitere 2 Stunden reagieren. Danach gibt man 2µl halbkonzentrierte Essigsäure zu und konzentriert im Vakuum. Nach Entsalzung über eine NAP-Säule™ (Pharmacia) wurde eine Butanol-Fällung durchgeführt. Man erhielt 8.1 OD (260) des gewünschten Oligonucleotid-Fluoresceindipivalat-Konjugats. ESI-MS: 4472.9 (berechnet MW 4471.6).

### Beispiel 11: Synthese von 5'-F9-A*T*G A C*G G A A*T*T*C

Das 5'-Aminolinker-A*T*G A C*G G A A*T*T*C-3' Oligomer wurde wie in Beispiel 9 beschrieben hergestellt. 10 OD (260) des 5'-Amino-Linker-Oligonucleotids wurden in 16 µl 0.2 M TBK-Puffer, 95 µl DMF gelöst und mit 25 µl des Aktivesters von Acetoxynaphtylcarbonsäure umgesetzt. Die Herstellung des Aktivesters erfolgte durch Mischen von 12.5 µl 0.2 M Acetoxynaphtylcarbonsäure (2.5 mg in 50 µl DMF) mit 12.5 µl TBTU ( 4 mg in 50 µl DMF) und einstündige Reaktion bei Umgebungstemperatur. Nach 17 Stunden Reaktion des Aminolinker-Oligonucleotids mit dem Aktivester gibt man 2µl halbkonzentrierte Essigsäure zu und konzentriert im Vakuum. Nach Entsalzung über eine NAP-Säule™ (Pharmacia) wurde eine Butanol-Fällung durchgeführt. Man erhielt 8.5 OD (260) des gewünschten Oligonucleotidacetoxynaphtyl-Konjugats. ESI-MS: 4158.2 (berechnet MW 4157.2).

### Beispiel 12: Synthese von 5'-F8-A*T*G A C*G G A A*T*T*C

Das 5'-Aminolinker-A*T*G A C*G G A A*T*T*C-3' Oligomer wurde wie in Beispiel 9 beschrieben hergestellt. 10 OD (260) des 5'-Amino-Linker-Oligonucleotids wurden in 16 µl 0.2 M TBK-Puffer, 95 µl DMF gelöst und mit 25 µl des Aktivesters von Acetoxycumarincarbonsäure umgesetzt. Die Herstellung des Aktivesters erfolgte durch Mischen von 12.5 µl 0.2 M Acetoxycumarincarbonsäure (2.7 mg in 50 µl DMF) mit 12.5 µl TBTU (4 mg in 50 µl DMF) und einstündige Reaktion bei Umgebungstemperatur. Nach 17 Stunden Reaktion des Aminolinker-Oligonucleotids mit dem Aktivester gibt man 2µl halbkonzentrierte Essigsäure zu und konzentriert im Vakkum. Nach Entsalzung über eine NAP-Säule™ (Pharmacia) wurde eine Butanol-Fällung durchgeführt. Man erhielt 8.0 OD (260) des gewünschten Oligonucleotidacetoxycumarin-Konjugats. ESI-MS: 4178.5 (berechnet MW 4175.2).

### Beispiel 13: Synthese von 5'-F3-(dA)ₙ dA*dA*dA ( n= 17, 47 und 77; F3 = FDA)

Die Oligonucleotide wurden ausgehend von einem CPG-Träger, der mit N6-Benzoyl-2'-deoxyadenosin über das 3'-Ende derivatisiert war, wie in Beispiel 1 beschrieben aufgebaut. Die Oxidationen nach den ersten beiden Kupplungen wurden mit Beaucage-Reagenz zur Einführung der Phosphorothioatreste (in der Sequenz mit * bezeichnet), alle restlichen Oxidationen wurden mit Jodwasser durchgeführt. Dann wurde der 5'-Amino-Modifier C6 wie in Beispiel 2 beschrieben aufgekuppelt. Nach der Entschützung mit conc. Ammoniak und 80% Essigsäure und Reinigung über ein präparatives Polyacrylamidgel erhielt man 2.95 OD (260) des 5'-Aminolinker-(dA)₁₇ dA*dA*dA, 4.9 OD (260) des 5'-Aminolinker-(dA)₄₇ dA*dA*dA und 5 OD (260) des 5'-Aminolinker-(dA)₇₇ dA*dA*dA. Die 5'-Amino-Linker-Oligonucleotide wurden jeweils in 8 µl 0.2 M TBK-Puffer, 62 µl DMF gelöst und mit 1.6 µmol FDA-Isothiocyanat umgesetzt. Nach 3 Stunden Reaktion gibt man weiteres FDA-Isothiocyanat zu und läßt noch 2 Stunden weiter reagieren. Dann gibt man 2µl halbkonzentrierte Essigsäure zu und konzentriert im Vakuum. Nach Entsalzung über eine NAP-Säule™ (Pharmacia) wurde eine Butanol-Fällung durchgeführt. Man erhält 1.5 OD (260) des 5'-F3-(dA)₁₇dA*dA*dA, 2.2 OD (260) des 5'-F3-(dA)₄₇dA*dA*dA und 0.9 OD (260) des 5'-F3-(dA)₇₇dA*dA*dA.

### Beispiel 14: Synthese des doppelt markierten Oligonucleotids 5'-Cy3-A*T*G A C*G G A A*T*T*C-C6-F3; F3=FDA

Das Oligonucleotid wurde ausgehend von einem CPG-Träger, der die Einführung eines C6-Aminolinkers am 3'-Ende erlaubt (Petrie et al. (1992) Bioconjugate Chem. 3, 85-87), wie in Beispiel 1 beschrieben aufgebaut. Die Oxidationen wurden mit Jodwasser bzw. Beaucage-Reagenz zur Einführung eines Phosphorothioatrestes (wenn * in der Sequenz) durchgeführt. Nach Abspaltung der letzten Dimethoxytritylschutzgruppe wurde das 5'-Ende des Oligonucleotids mit einem Cy3-CE Phosphoramidit (Firma Glen Research, Sterlin, VA; Katalog-Nr. 10-5913-xx) umgesetzt und mit Jodwasser oxidiert. Nach der Entschützung mit conc. Ammoniak (2 Stunden bei 70°C) erhielt man 64 OD (260) des Rohproduktes. Nach der Reinigung über ein präparatives Polyacrylamidgel erhält man 3.8 OD des des 5'-Cy3-A*T*G A C*G G A A*T*T*C-C6-aminolink-3'. Davon werden 3.5 OD (260) in 8 µl 0.2 M TBK-Puffer, 62 µl DMF gelöst und mit 1.6 µmol FDA-Isothiocyanat umgesetzt. Nach 3.5 Stunden Reaktion des Aminolinker-Oligonucleotids mit dem Hydroxysuccinimid gibt man 1µl halbkonzentrierte Essigsäure zu und konzentriert im Vakuum. Nach Entsalzung über eine NAP-Säule™ (Pharmacia) wurde eine Butanol-Fällung durchgeführt. Man erhielt 3.5 OD (260) des gewünschten doppelt markierten Oligonucleotids 5'-Cy3-A*T*G A C*G G A A*T*T*C-C6-F3. ESI-MS: 4926.2 (berechnet MW 4927.1).

### Beispiel 15: Synthese von 5'-F3-A*T*G A C*G G A A*T*T*C; F3=FDA

Das 5'-Aminolinker-A*T*G A C*G G A A*T*T*C-3' Oligomer wurde wie in Beispiel 9 beschrieben hergestellt. 10 OD (260) des 5'-Amino-Linker-Oligonucleotids wurden in 16 µl 0.2 M TBK-Puffer, 95 µl DMF gelöst und mit 25 µl des FDA-Isothiocyanat (5 mg in 100 µl DMF) umgesetzt. Nach 3 Stunden Reaktion des Aminolinker-Oligonucleotids mit dem Isothiocyanat gibt man nochmals 5 µl des Isothiocyanats zu und lässt weitere 2 Stunden reagieren. Danach gibt man 2µl halbkonzentrierte Essigsäure zu und konzentriert im Vakuum. Nach Entsalzung über eine NAP-Säule™ (Pharmacia) wurde eine Butanol-Fällung durchgeführt. Man erhielt 8.5 OD (260) des gewünschten Oligonucleotid-Fluoresceindiacetat-Konjugats. ESI-MS: 4418.4 (berechnet MW 4418.5).

### Beispiel 16: Vergleich der zellulären Aufnahme von Oligonucleotid-Konjugaten unterschiedlicher Länge 5'-F3-(dA)ₙ dA*dA*dA ( n= 17, 47 und 77; F3 = FDA)

Die Bestimmung der zellulären Aufnahme der Konjugate unterschiedlicher Länge und Molekulargewichts aus Beispiel 12 wurde im Prinzip wie in Beispiel 7 beschrieben mit REH-Zellen durchgeführt. Die Quantifizierung erfolgte mit Hilfe eines Durchflußzytometers. Nach 60 Minuten sind die relativen Fluoreszenzsignale für 5'-F3-(dA)₁₇dA*dA*dA ("A20") gleich 49, für 5'-F3-(dA)₄₇dA*dA*dA ("A50") gleich 34, und für 5'-F3-(dA)₇₇dA*dA*dA ("A80") gleich 91. Das FDA-Konjugat mit dem größten Oligonucleotid-Teil, bestehend aus insgesamt 80 Nucleotiden, wurde überraschenderweise am effektivsten von den REH-Zellen aufgenommen.

### Beispiel 17: Vergleich der zellulären Aufnahme von Oligonucleotid-Konjugaten unterschiedlicher Derivatisierung

Eine mit Fluorescein-Diacetat (FDA) verwandte Verbindung ist das Fluorescein-Dipivalat, das sich durch eine erhöhte Stabilität der Estergruppen auszeichnet. Die entsprechenden Oligonukleotid-Konjugate wurden am Durchflußzytometer auf ihre Aufnahme untersucht. Die höhere Stabilität des Pivalats gegenüber Alkali und Esterasen führt zu einer geringeren Aufnahme des entsprechenden Oligonucleotid-Konjugats. So ist die gemessene relative Fluoreszenz für das Fluorescein-Diacetat-Konjugat nach 60 Minuten gleich 195, während sie für das entsprechende Fluorescein-Dipivalat-Konjugat nur 55 beträgt.

### Beispiel 18: Untersuchung der zellulären Aufnahme des zweifach markierten Oligonucleotid-Konjugats 5'-Cy3-A*T*G A C*G G A A*T*T*C-C6-FDA aus Beispiel 13.

Am FACScan zeigte das Cy3-Oligonucleotid-F3-Konjugat eine schnelle Aufnahme in REH-Zellen. Die Behandlung der Zellen mit einem Oligonukleotid-Konjugat / Cellfectin-Komplex gibt eine ähnliche, aber deutlich ungleichmäßigere Aufnahme. Dabei überlagert der Effekt des FDA-Konjugates deutlich den des Cellfectin-Oligonukleotidkomplexes.

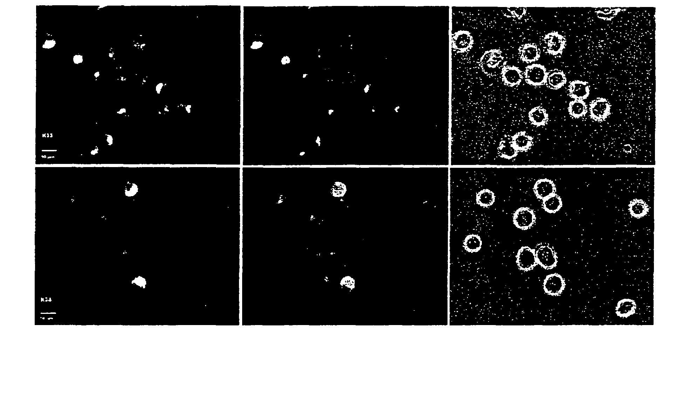

Weiterhin sollte die Ko-Lokalisation der beiden unterschiedlichen Marker-Gruppen am Oligonucleotid bei der Inkubation mit Zellen untersucht werden, um auszuschließen, dass die gemessene Fluoreszenz nur auf dem abgespaltenen FDA bzw. Fluorescein beruht. Das Cy3-Oligonucleotid-F3-Konjugat enthielt den Cyaninfarbstoff am 5'-Ende und das FDA am 3'-Ende kovalent gebunden. Abbildung 9 zeigt die Grün- und Rot-Fluoreszenz nach 4-stündiger Inkubation der REH-Zellen mit dem Cy3-Oligonucleotid-F3-Konjugat. Da eine Ko-Lokalisation der beiden Marker in den Zellen zu beobachten ist, muss man davon ausgehen, daß das Oligonukleotid in intakter Form aufgenommen wurde. Lediglich die Acetylgruppen des FDA-Teils wurden hydrolysiert, vermutlich durch Esterasen, da der nichtfluoreszierende FDA-Rest offensichtlich in den fluoreszierenden Fluorescein-Rest übergeführt wurde. Die Aufnahme des Cy3/FDA-Konjugates erfolgte sehr schnell, da die beiden Marker bereits 7 min nach Zugabe nachgewiesen werden konnten.

Das Oligonucleotid-FDA-Konjugat KO_1 aus Beispiel 4 wurde bei vier Konzentrationen auf die antiproliferative Wirkung von A549 Tumorzellen getestet. Es hemmte die Proliferation ohne Zugabe eines Penetrationsverstärkers. Das entsprechende Oligonucleotid ohne F3-Konjugat (ON1) hemmt die Proliferation nur nach Komplexbildung mit einem Penetrationsverstärker (CellFectin, Fa. Gibco-BRL).Die Ergebnisse sind in Tabelle 4 dargestellt.

**Tabelle 1: Fluoreszenzmikroskopische Untersuchung der Aufnahme von FDA-markierten Oligonukleotiden(Konjugat Oligonukleotid-FDA) in Säugerzellen.**

| Säugerzellen: Bezeichnung | Fluoreszenz nach 5 min | | Fluoreszenz nach 20 min | | Fluoreszenz nach 60 min | | Fluoreszenz nach 120 min | |
|---|---|---|---|---|---|---|---|---|
| der Zellinie | A | B | A | B | A | B | A | B |
| REH | + | - | + | - | ++ | - | ++ | * |
| K562 | | | (+) | | + | | + | |
| Lu 18 | | | | | (+) | | + | |
| KB3-1 | | | | | + | | + | |
| Ptk 2 | | | | | (+) | | + | |

| | | | | | | | | |
|---|---|---|---|---|---|---|---|---|
| A: Inkubation mit FDA-markierten Oligonukleotiden KO_1 und KO_3 B: Inkubation mit Fluorescein-markierten Oligonukleotiden KO_2 und KO_4 (+) schwache Aufnahme + mittelstarke Aufnahme ++ sehr starke Aufnahme - keine Aufnahme * Aufnahme nur in geschädigte Zellen | | | | | | | | |

**Tabelle 2: Fluoreszenzmikroskopische Untersuchung der Aufnahme von FDA-markierten Oligonukleotiden in Insektenzellen (Legende s. Tabelle 1).**

| Insektenzellen | Fluoreszenz nach 20 min | | nach 60 min | | nach 120 min | |
|---|---|---|---|---|---|---|
| | A | B | A | B | A | B |
| SF9 Zellen | + | - | ++ | - | ++ | - |

**Tabelle 3: Fluoreszenzmikroskopische Untersuchung der Aufnahme von FDA-markierten Oligonukleotiden in verschiedene Organismen.**

| Organismus | Fluoreszenz nach 20 min | | Fluoreszenz nach 60 min | | Fluoreszenz nach 120 min | |
|---|---|---|---|---|---|---|
| | A | B | A | B | A | B |
| Bac. subtilis (6633) ^{#} - | | - | - | - | + | - |
| L. bulgaricus ^{#} | - | - | + | - | + | - |
| E. coli (K12) ^{#} | - | - | + | - | + | - |
| Yarrowia lipolytica ^{#} (Wildform H 222) | - | - | - | - | + | - |
| Saccaromyces cerevisiae ^{#} | - | - | - | - | + | - |
| Fusarium culmorum -Sporen (JP15, Pilz) | (+) | - | + | - | + | - |
| Reticulomyxa filosa-Cysten (Süßwasseramoebe) | - | - | ++ besond. Nuklei | - | ++ | - |
| Haematococcus pluvialis (Schneealge, Flagellat) | - | - | + | - | + | - |
| Chlorogonium sp. (Grünalge, Flagellat) | - | - | + | - | + | - |
| Dunaliella salina (Meeres-Diatomee) | - | - | + | - | + | - |

| | | | | | | |
|---|---|---|---|---|---|---|
| A: Inkubation mit FDA-markierten Oligonukleotiden KO_1 und KO_3 B: Inkubation mit Fluorescein-markierten Oligonukleotiden KO_2 und KO_4 (legende zur bewertung s. Tabelle 1) # Aufnahme erfolgt nur in Teil der Zellen dieser schnellteilenden Organsimen | | | | | | |

**Tabelle 4: Ergebnisse aus Beispiel 8.**

| Substanz | Zelldichte | % Inhibition |
|---|---|---|
| ohne | 5.96 | - |
| FDA | 6.05 | - 1.5 |
| 100 nM KO_1 | 5.65 | 5.2 |
| 200 nM KO_1 | 5.3 | 11.1 |
| 500 nM KO_1 | 5.03 | 15.6 |
| 1000 nM KO_1 | 4.16 | 30.2 |

**Tabelle 5: Längenabhängigkeit der Transfektion von FDA-Konjugaten (A20, A50, A80 aus Beispiel 15).**

| Zeit [min] | Fluoreszenz | Fluoreszenz | Fluoreszenz |
|---|---|---|---|
| | A20 | A50 | A80 |
| 0 | 0,0103 | 0,0103 | 0,0103 |
| 10 | 11,71 | 8,9 | 43,31 |
| 20 | 39,68 | 28,06 | 88,36 |
| 30 | 48,59 | 34,38 | 91,28 |
| 40 | 54,7 | 38,75 | 92,35 |
| 50 | 58,66 | 41,64 | 93,19 |
| 60 | 62,13 | 44,33 | 93,62 |

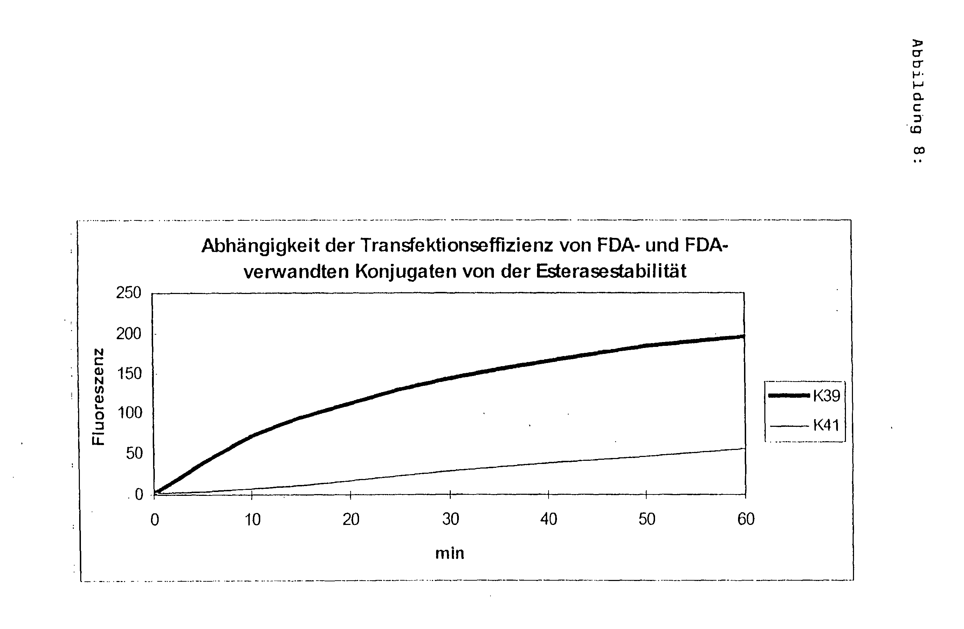

**Tabelle 6: Vergleich der Zellaufnahme von Fluorescein-Diacetat- und Fluorescein-Dipivalat Oligonukleotid Konjugaten (Beispiel 16, Abbildung 8).**

| Zeit [min] | Fluoreszenz | Fluoreszenz |
|---|---|---|
| | K39 | K41 |
| | | |
| 0 | 2,66 | 2,75 |
| 10 | 72,37 | 8,49 |
| 20 | 112,34 | 18,17 |
| 30 | 142,97 | 28,67 |
| 40 | 164,64 | 38,34 |
| 50 | 184,96 | 46,62 |
| 60 | 195,57 | 55,26 |
| 70 | | |
| 80 | 218,12 | 68,02 |
| 90 | | |
| 100 | 231,04 | 83,7 |
| 110 | | |
| 120 | 253,84 | 97 |

## Patentansprüche

1. Verwendung eines Arylrestes der Formeln F1 und F3 bis F11, mit 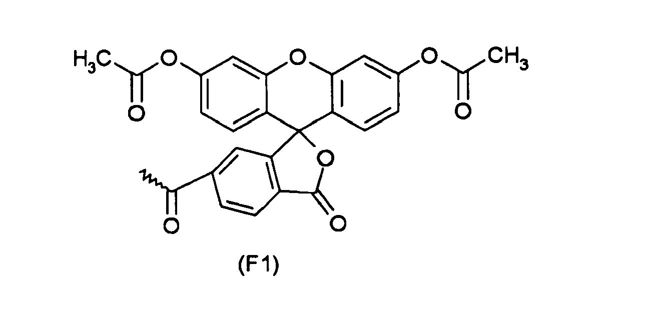 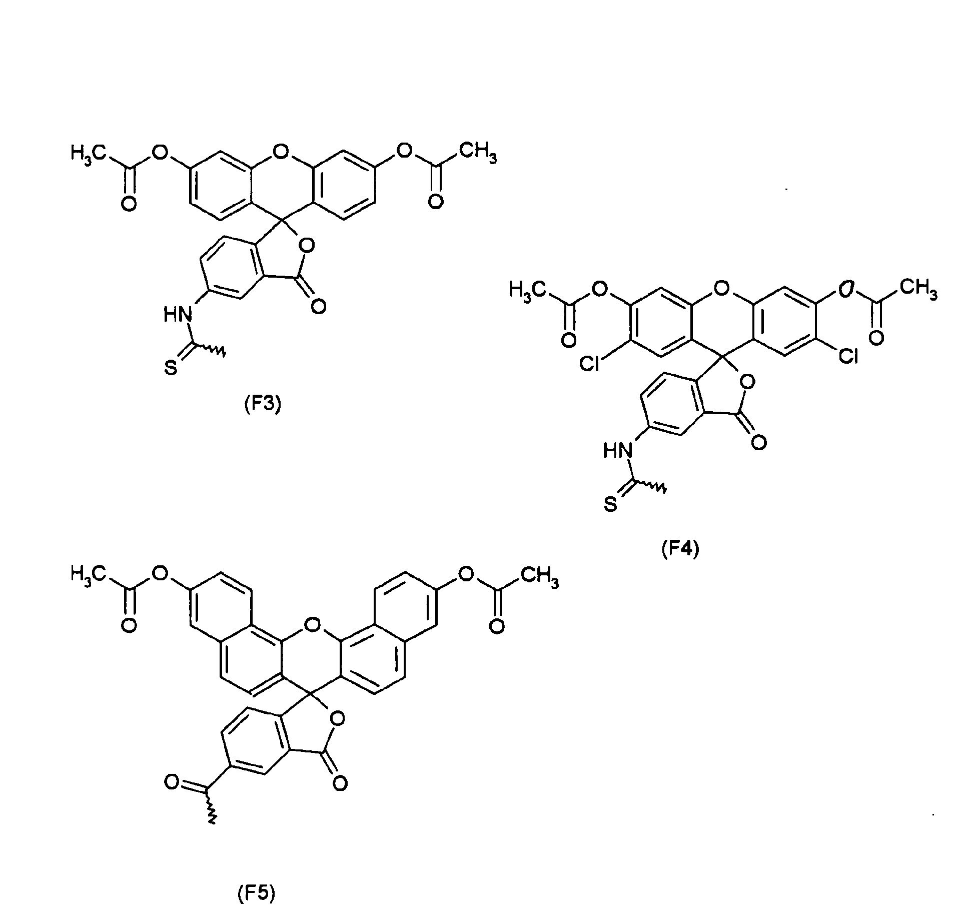 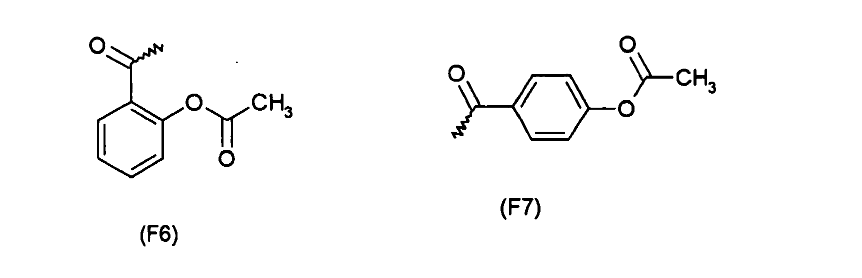 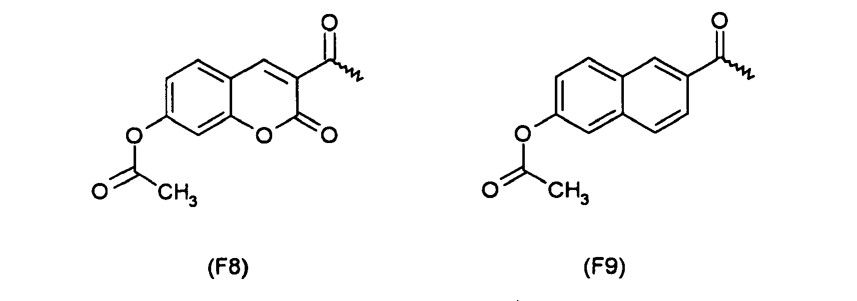 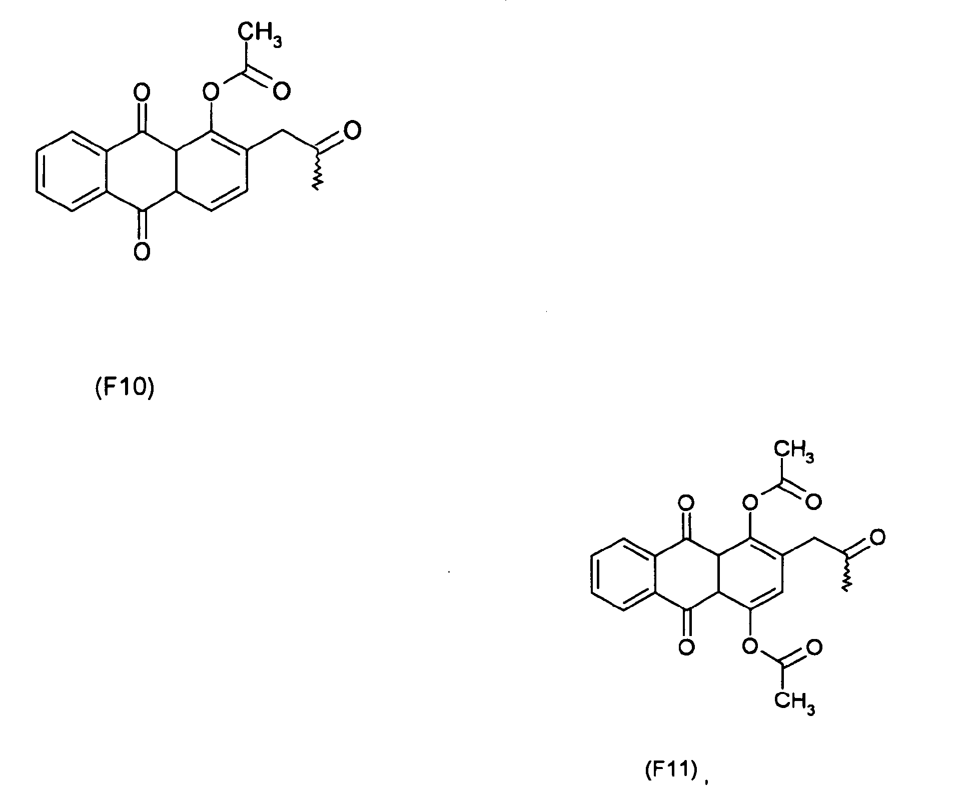 der an ein zu transportierendes Molekül ausgewählt aus einem Polynukleotid, Oligonukleotid oder Mononukleotid gebunden ist, zum Transport dieses Moleküls über eine biologische Membran in vitro.

2. Verwendung eines Arylrestes der Formeln F1 und F3 bis F11, mit 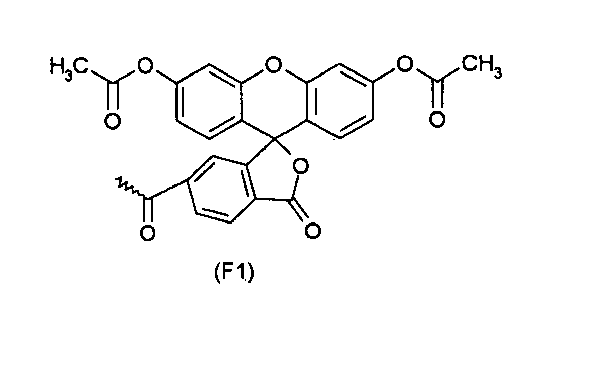 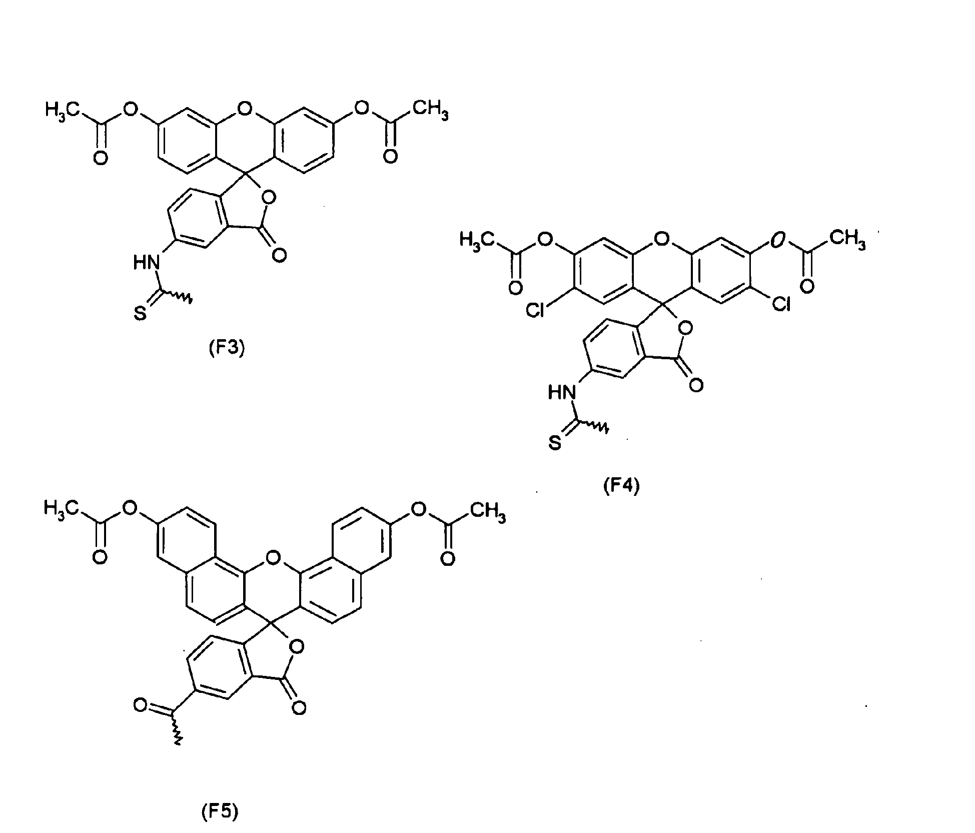 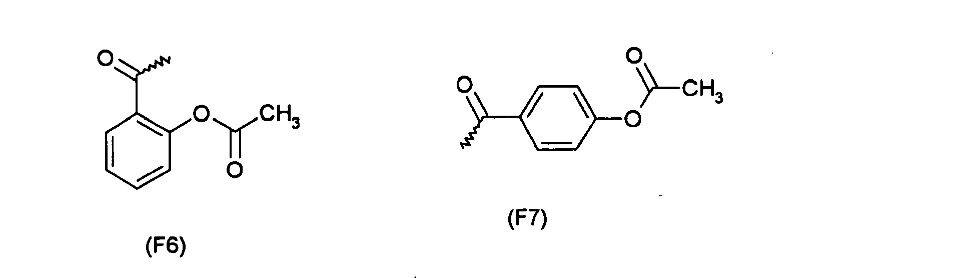 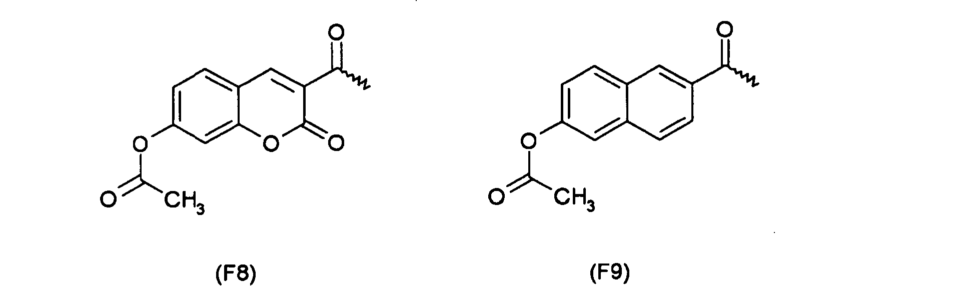 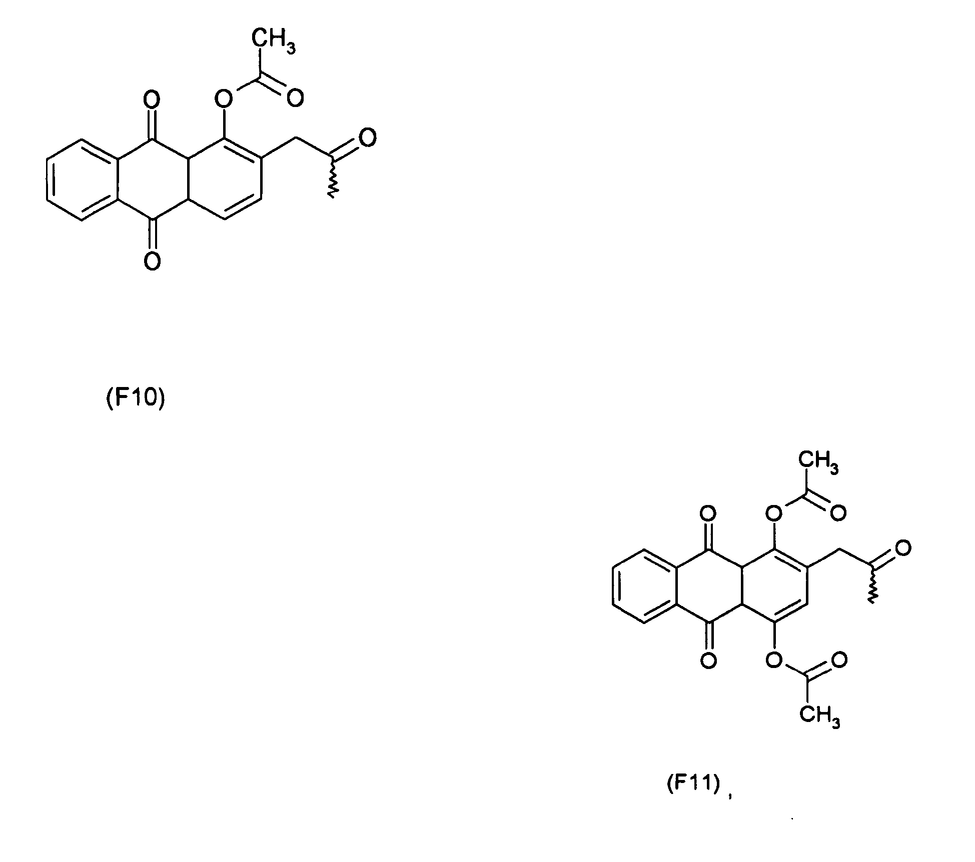 der an ein zu transportierendes Molekül ausgewählt aus einem Polynukleotid, Oligonukleotid oder Mononukleotid gebunden ist, zur Herstellung eines Arzneimittels zur Prävention und/oder Behandlung von Krankheiten, die mit der Expression bzw. einer Überexpression bestimmter Gene einhergehen, und/oder eines Diagnostikums für die Diagnose bzw. Früherkennung solcher Krankheiten.

3. Verwendung gemäß Anspruch 1 oder 2,
wobei der/die Aryl-Rest(e) an das 5'-Ende, 3'-Ende, die heterozyklische Base, den Zucker oder die Internukleosidbrücke, aber auch über nichtnukleotidische Bausteine gebunden ist/sind.

4. Verfahren zum Transport eines Moleküls über eine Membran in vitro, wobei ein Arylrest, wie in einem der Ansprüche 1-3 beschrieben mit der an ein zu transportierendes Molekül ausgewählt aus einem Polynukleotid, Oligonukleotid oder Mononukleotid gebunden ist mit der Membran inkubiert wird.

5. Verfahren zum Transport eines Moleküls in eine Zelle in vitro, wobei ein Arylrest, wie in einem der Ansprüche 1-3 beschrieben mit der an ein zu transportierendes Molekül ausgewählt aus einem Polynukleotid, Oligonukleotid oder Mononukleotid gebunden ist mit der Zelle inkubiert wird, woraufhin das Konjugat in die Zelle transportiert wird, ohne daß der Aryl-Rest abgespalten wird.

6. Verfahren nach Anspruch 5, wobei die Zelle eine eukaryotische oder eine prokaryotische Zelle ist.

7. Verfahren nach Anspruche 5 oder 6, wobei die Zelle eine Bakterienzelle, Hefezelle oder eine Säugetierzelle ist.

8. Verfahren nach einem oder mehreren der Ansprüche 5 bis 7, wobei die Zelle eine humane Zelle ist.

9. Verfahren nach einem oder mehreren der Ansprüche 5 bis 8, wobei die Zelle eine Tumorzelle ist.

## Claims

1. The use of an aryl radical of the formulae F1 and F3 to F11, 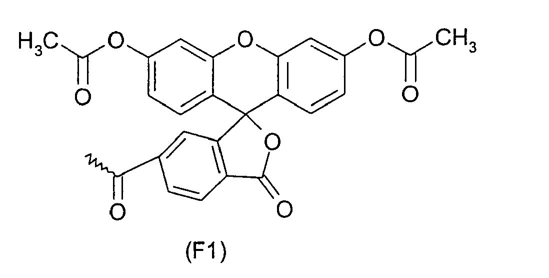 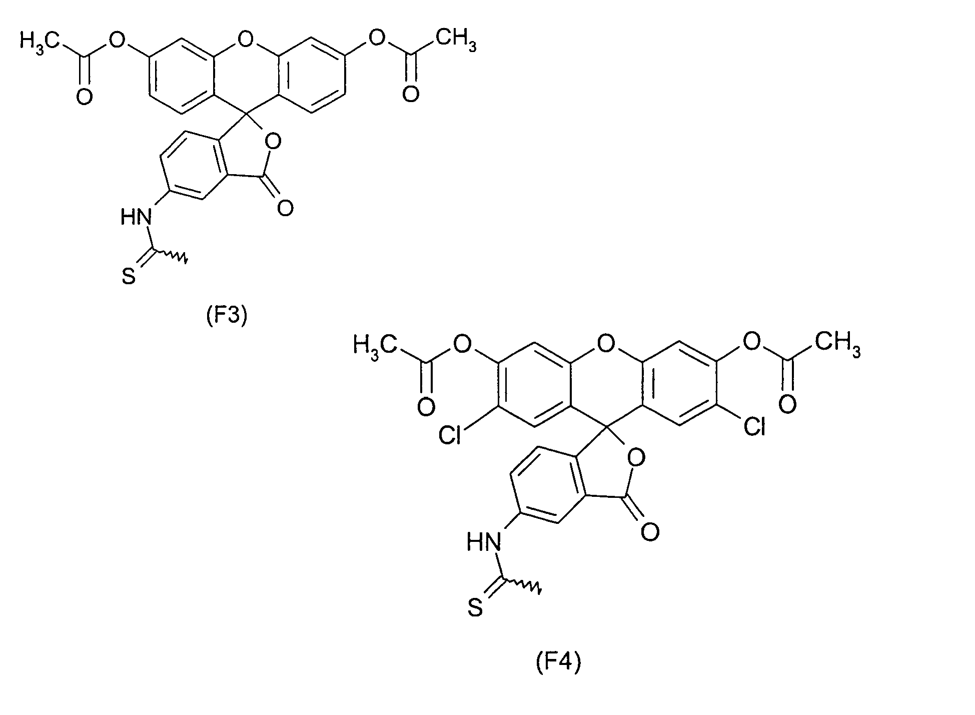 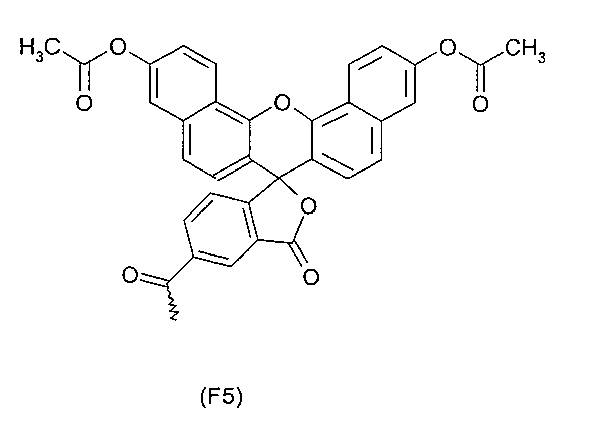 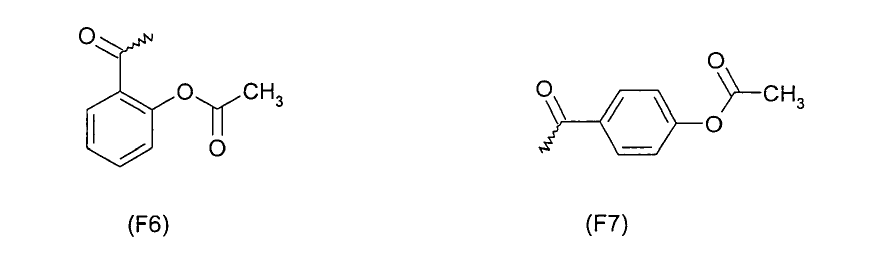 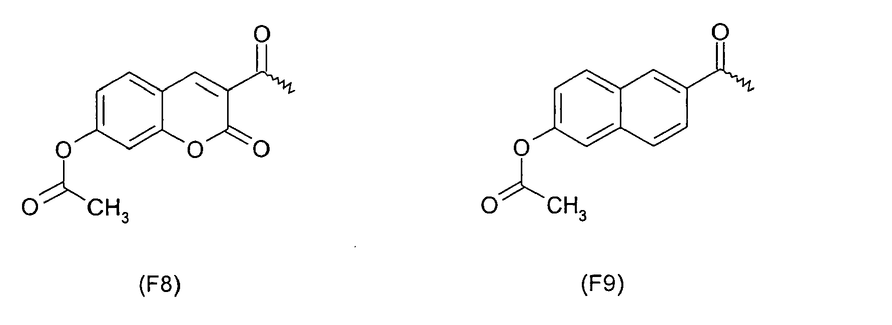 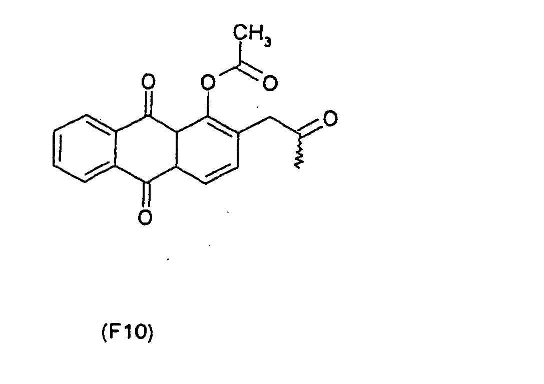 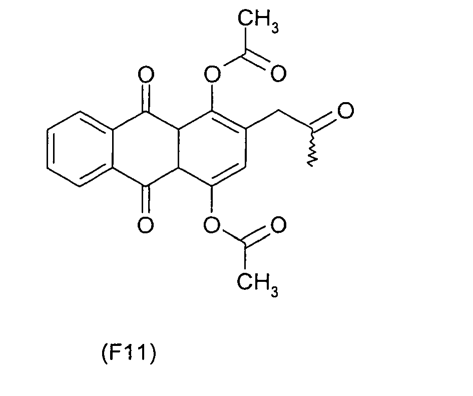 to which a molecule to be transported selected from the group consisting of a polynucleotide, an oligonucleotide and a mononucleotide is attached, for transporting this molecule across a biological membrane in vitro.

2. The use of an aryl radical of the formulae F1 and F3 to F11, 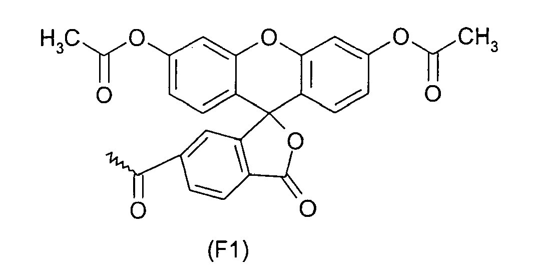 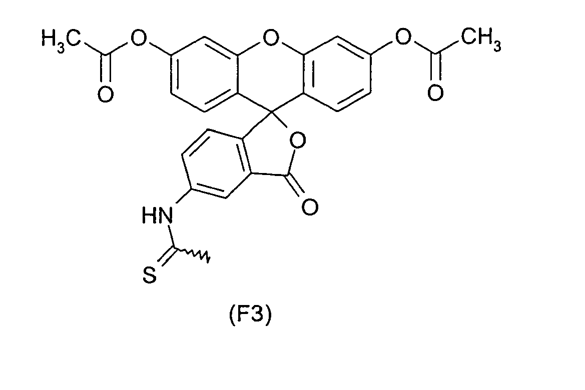 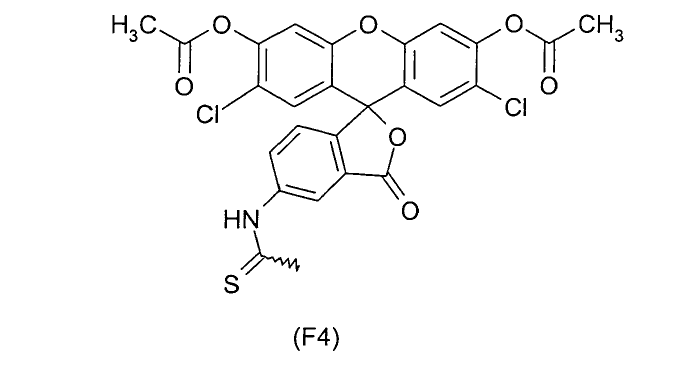 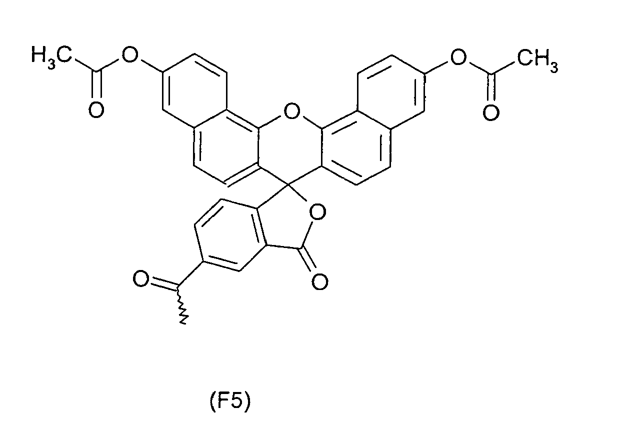 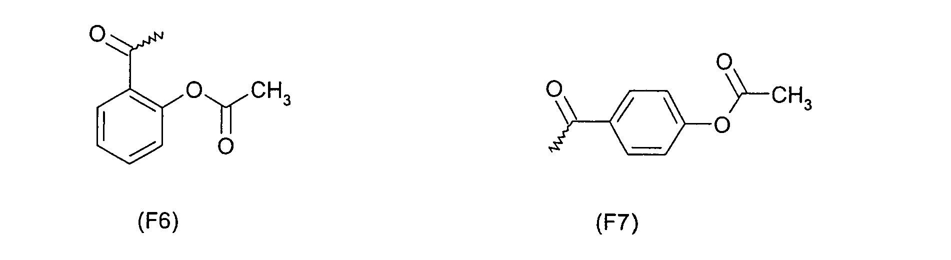 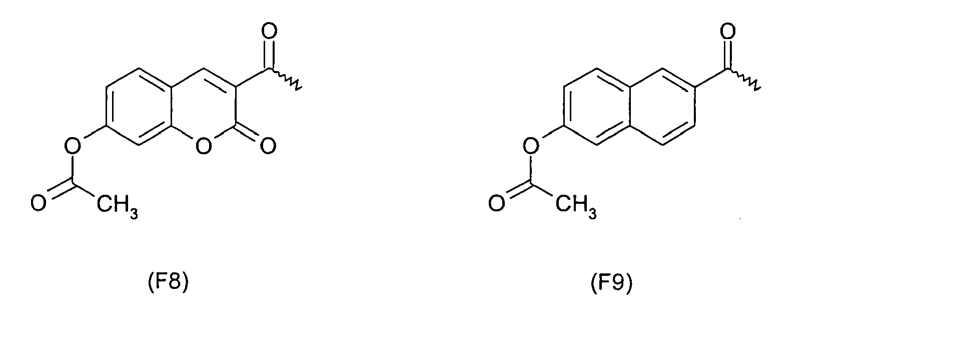 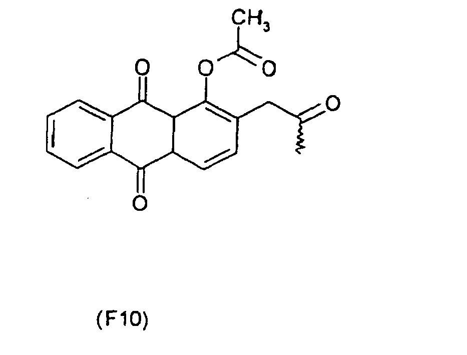 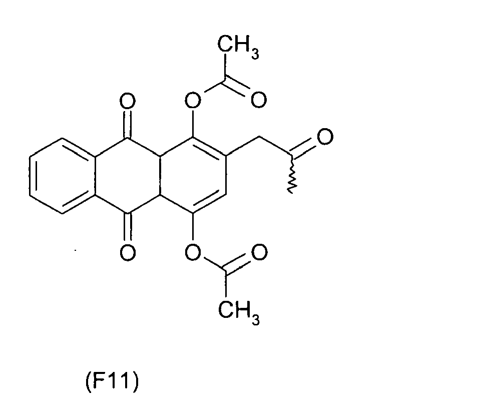 to which a molecule to be transported selected from the group consisting of a polynucleotide, an oligonucleotide and a mononucleotide is attached, for preparing a medicament for the prevention and/or treatment of diseases associated with the expression or an overexpression of certain genes, and/or a diagnostic for the diagnosis or early identification of such diseases.

3. The use as claimed in claim 1 or 2,
where the aryl radical (s) is (are) attached to the 5'-terminal, the 3'-terminal, the heterocyclic base, the sugar or the internucleoside bridge, and may also be attached via non-nucleotidic building blocks.

4. A method for transporting a molecule across a membrane in vitro, where an aryl radical as described in any of claims 1-3 to which a molecule to be transported selected from the group consisting of a polynucleotide, an oligonucleotide or a mononucleotide is attached, is incubated with the membrane.

5. A method for transporting a molecule into a cell in vitro, where an aryl radical as described in any of claims 1-3 to which a molecule to be transported selected from the group consisting of a polynucleotide, an oligonucleotide or a mononucleotide is attached, is incubated with the cell, whereupon the conjugate is transported into the cell without the aryl radical being cleaved off.

6. The method as claimed in claim 5, wherein the cell is a eukaryotic or a prokaryotic cell.

7. The method as claimed in claim 5 or 6, wherein the cell is a bacterial cell, yeast cell or a mammalian cell.

8. The method as claimed in one or more of claims 5 to 7, wherein the cell is a human cell.

9. The method as claimed in one or more of claims 5 to 8, wherein the cell is a tumour cell.

## Revendications

1. Utilisation d'un radical aryle de formules F1 et F3 à F11, 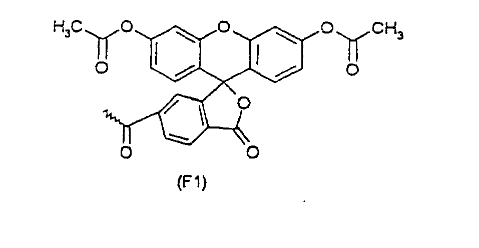 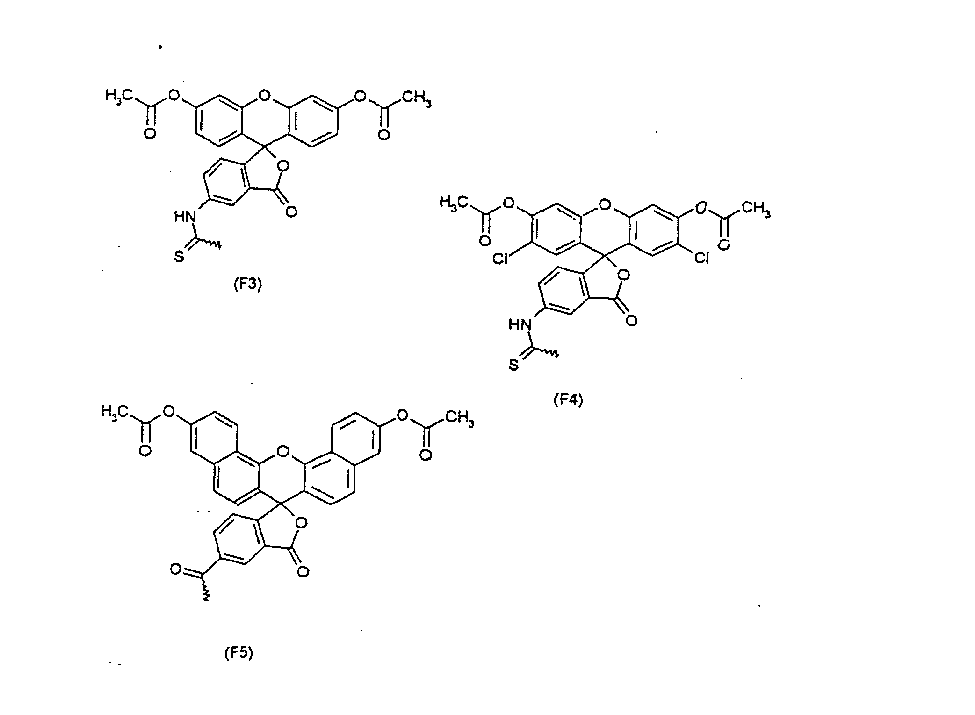 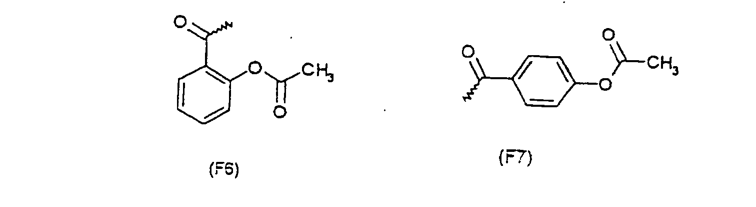 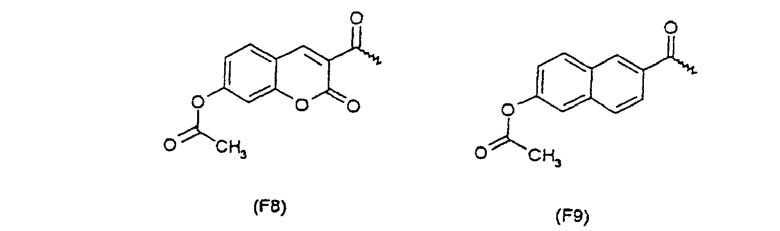 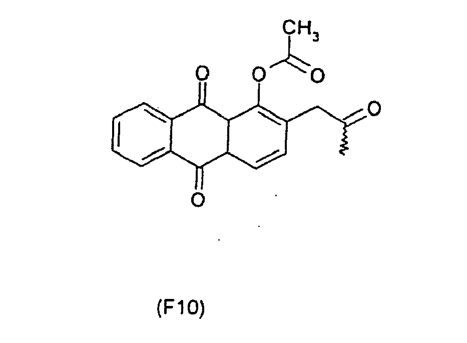 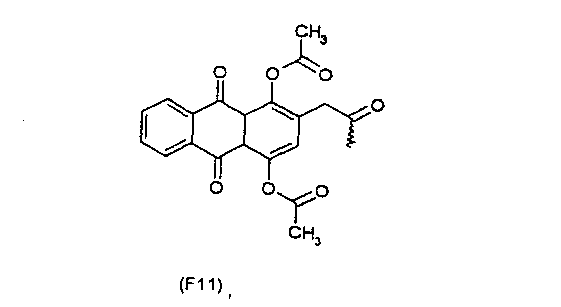 qui est lié à une molécule à transporter, choisie parmi un polynucléotide, un oligonucléotide ou un mononucléotide, pour le transport de cette molécule à travers une membrane biologique in vitro.

2. Utilisation d'un radical aryle de formules F1 et F3 à F11, 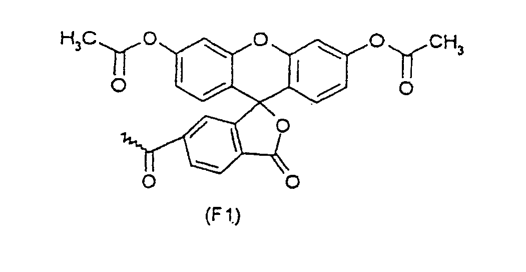 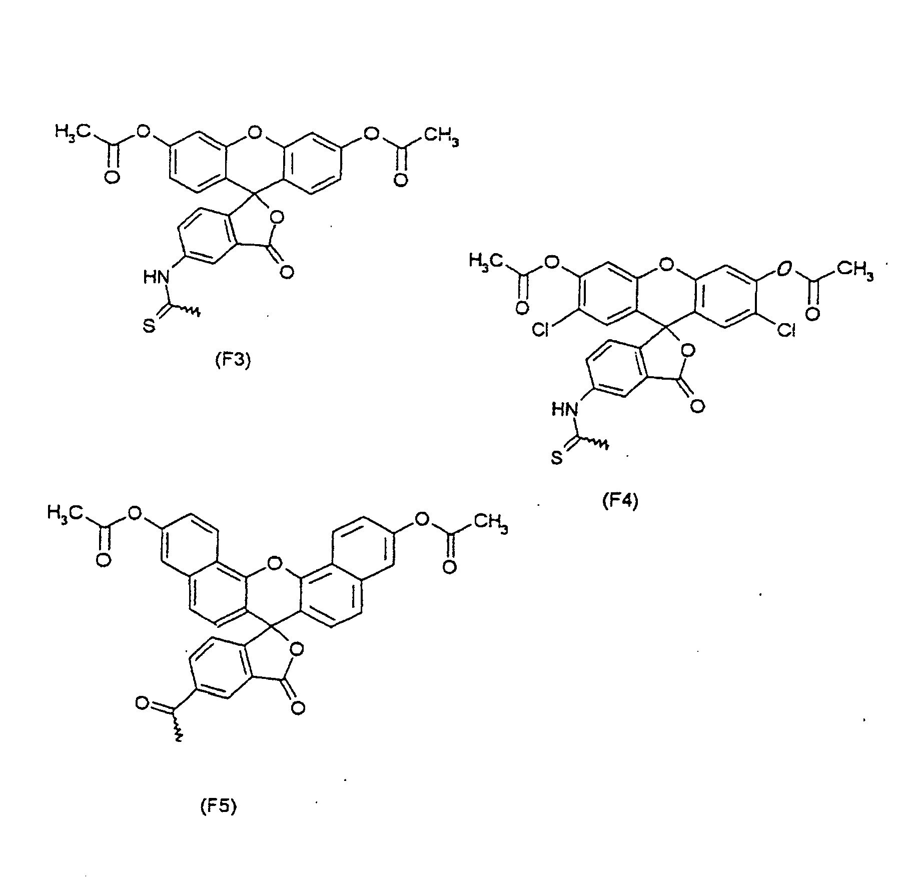 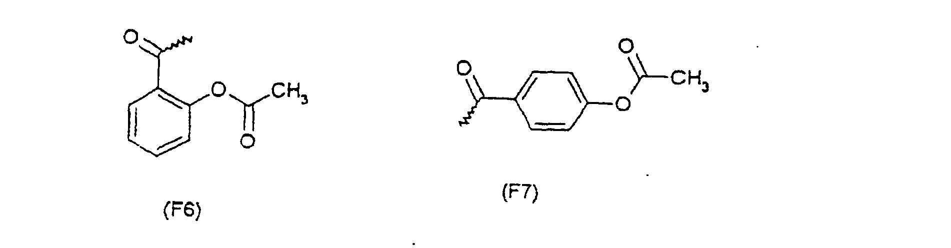 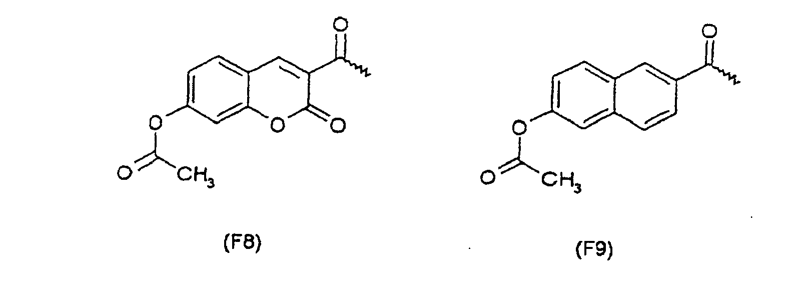 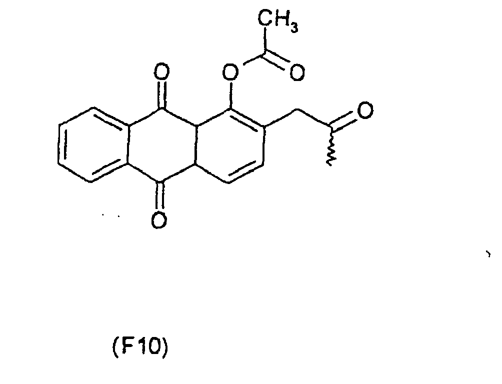 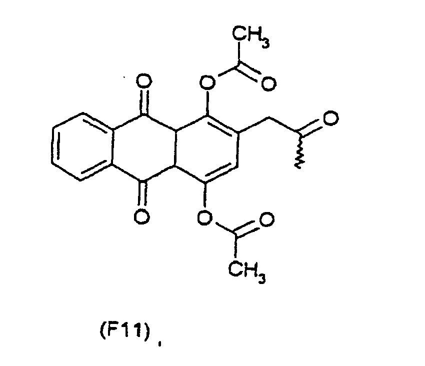 où qui est lié à une molécule à transporter, choisie parmi un polynucléotide, un oligonucléotide ou un mononucléotide, pour la fabrication d'un médicament destiné à la prévention et/ou au traitement de maladies qui s'accompagnent de l'expression ou d'une surexpression de certains gènes, et/ou d'un agent de diagnostic pour le diagnostic ou le dépistage précoce de telles maladies.

3. Utilisation selon la revendication 1 ou 2, dans laquelle le(s) radical/radicaux est/sont lié(s) à l'extrémité 5', à l'extrémité 3', à la base hétérocyclique, au sucre ou au pont inter-nucléoside, mais également par l'intermédiaire de composants non nucléotidiques.

4. Procédé pour le transport d'une molécule à travers une membrane in vitro, dans lequel on met à incuber avec la membrane un radical aryle tel que décrit dans l'une quelconque des revendications 1 à 3, auquel est liée une molécule à transporter, choisie parmi un polynucléotide, un oligonucléotide ou un mononucléotide.

5. Procédé pour le transport d'une molécule dans une cellule in vitro, dans lequel on met à incuber avec la cellule un radical aryle tel que décrit dans l'une quelconque des revendications 1 à 3, auquel est liée une molécule à transporter, choisie parmi un polynucléotide, un oligonucléotide ou un mononucléotide, à la suite de quoi le conjugué est transporté dans la cellule, sans que le radical aryle soit séparé.

6. Procédé selon la revendication 5, dans lequel la cellule est une cellule eucaryote ou une cellule procaryote.

7. Procédé selon la revendication 5 ou 6, dans lequel la cellule est un cellule bactérienne,une cellule de levure ou une cellule mammalienne.

8. Procédé selon une ou plusieurs des revendications 5 à 7, dans lequel la cellule est une cellule humaine.

9. Procédé selon une ou plusieurs des revendications 5 à 8, dans lequel la cellule est une cellule tumorale.
